(19) Europäisches Patentamt / European Patent Office / Office européen des brevets

(11) **EP 3 357 466 B1**

(12) **EUROPEAN PATENT SPECIFICATION**

(45) Date of publication and mention
of the grant of the patent:
**08.05.2019 Bulletin 2019/19**

(21) Application number: **16850770.5**

(22) Date of filing: **23.06.2016**

(51) Int Cl.:
*A61F 13/53* (2006.01)          *A61F 13/534* (2006.01)
*A61F 13/537* (2006.01)

(86) International application number:
**PCT/JP2016/068718**

(87) International publication number:
**WO 2017/056593 (06.04.2017 Gazette 2017/14)**

(54) **ABSORBENT BODY FOR ABSORBENT ARTICLE**

SAUGFÄHIGER KÖRPER FÜR EINEN SAUGFÄHIGEN ARTIKEL

CORPS ABSORBANT POUR ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2015 JP 2015195123**

(43) Date of publication of application:
**08.08.2018 Bulletin 2018/32**

(73) Proprietor: **Unicharm Corporation
Ehime 799-0111 (JP)**

(72) Inventors:
• **TANGE, Satoru
Kanonji-shi
Kagawa 769-1602 (JP)**

• **GODA, Hiroki
Kanonji-shi
Kagawa 769-1602 (JP)**

(74) Representative: **Staeger & Sperling
Partnerschaftsgesellschaft mbB
Sonnenstraße 19
80331 München (DE)**

(56) References cited:
**WO-A1-2014/140751       WO-A1-2015/076136
JP-A- 2012 105 962        JP-A- 2013 102 930
JP-A- 2013 180 171        JP-A- 2014 094 200
US-A1- 2008 312 628**

**Description**

Technical Field

The present disclosure relates to an absorbent body for an absorbent article.

Background Art

In an absorbent article such as a disposable diaper, etc., an absorbent body which absorbs liquid, such as body fluid, etc., is generally disposed in a wide range of the absorbent article, with a liquid supply point (an excretory opening contact region) of the liquid as the center. In such an absorbent article in which the absorbent body is disposed in the wide range thereof, for the purpose of increasing the use efficiency of the absorbent body, it is known to dispose a diffusion sheet between the liquid permeable sheet and the absorbent core, which diffuses the absorbed liquid from the liquid supply point in a plane direction of the absorbent article.

For example, paragraph [0024] of Patent Literature 1 discloses that a diffusion layer 6 which has a body fluid diffusing property can be provided between, for example, the top surface layer 2 (or the back surface layer 3) and a portion including the polymer absorbing agents. Further, paragraph [0024] of Patent Literature 1 also discloses a fiber aggregation body which is formed by laminating, as a diffusion layer, pulp fibers on a web of rayon fibers and entangling the laminated layers by water jets.

Citation List

Patent Literature

PTL 1: Japanese Unexamined Patent Publication No. 2004-298384

Summary of Invention

Technical Problem

However, while the diffusion sheet has an excellent ability to diffuse the absorbed liquid in a plane direction of the absorbent article, the diffusion sheet often has insufficient ability to transfer the absorbed liquid in a thickness direction of the absorbent article, and there are many cases in which the absorbed liquid stays in the diffusion sheet and the absorbent article including the diffusion sheet has insufficient absorbability.

Accordingly, the object of the present disclosure is to provide an absorbent body which is excellent in diffusing liquid in a plane direction and in transferring the liquid in a thickness direction, when the liquid is repeatedly absorbed.

Solution to Problem

The present inventors have found that an absorbent body for an absorbent article, comprising an absorbent core which includes a polymer absorbing agent and further comprising a core wrapping sheet, wherein the core wrapping sheet includes (i) a first hydrophilic fiber containing layer which is adjacent to the absorbent core and includes first hydrophilic fibers with an average fiber length of 25 mm to 75 mm, (ii) a pulp fiber containing layer which is adjacent to the first hydrophilic fiber containing layer and includes pulp fibers, and (iii) a second hydrophilic fiber containing layer which is adjacent to the pulp fiber containing layer and includes second hydrophilic fibers with an average fiber length of 25 mm to 75 mm, and a portion of the pulp fibers in the pulp fiber containing layer penetrates the first hydrophilic fiber containing layer and is in contact with the absorbent core, is the solution to the problem.

Effect of Invention

The absorbent body of the present disclosure is excellent in diffusing liquid in a plane direction and in transferring the liquid in a thickness direction, when the liquid is repeatedly absorbed.

Brief Description of Drawing

FIG. 1 is an expanded view of an absorbent article 1 which includes an absorbent body 7 according to a first embodiment.

FIG. 2 is an end view of the absorbent body 7 at a II - II end surface of FIG. 1.

FIG. 3 is a view for explaining the effect of the present disclosure.

FIG. 4 is another view for explaining the effect of the present disclosure.

FIG. 5 is still another view for explaining the effect of the present disclosure.

FIG. 6 is still another view for explaining the effect of the present disclosure.

FIG. 7 is still another view for explaining the effect of the present disclosure.

FIG. 8 is a view for explaining a manufacturing method of a core wrapping sheet.

Description of Embodiments

[Definitions]

• "Average fiber length"

In the present disclosure, the average fiber length of fibers other than pulp fibers, for example, first hydrophilic fibers and second hydrophilic fibers, is measured in accordance with "A7.1.1 Method A (Standard method) A method of measuring a length of individual fibers on a scaled glass plate" of "A7.1 Measurement of a fiber length" of JIS L 1015:2010 Annex A.

Incidentally, the above mentioned method is a test method which corresponds to ISO 6989 issued in 1981.

• "Average fiber length"

In the present disclosure, the average fiber length of pulp fibers means the load-weighted average fiber length, and means the value L (w) which is measured by "kajaaniFiberLab fiber properties (off-line)" manufactured by Metso Automation Inc.

• "Cellulose based fiber"

In the present disclosure, "a cellulose based fiber" means a fiber which is selected from a group consisted of a regenerated cellulose fiber, a purified cellulose fiber, and a semi-synthetic cellulose fiber.

The present disclosure relates to the following aspects.

[Aspect 1]

An absorbent body for an absorbent article, comprising an absorbent core which includes a polymer absorbing agent and further comprising a core wrapping sheet, wherein

the core wrapping sheet includes (i) a first hydrophilic fiber containing layer which is adjacent to the absorbent core and includes first hydrophilic fibers with an average fiber length of 25 mm to 75 mm, (ii) a pulp fiber containing layer which is adjacent to the first hydrophilic fiber containing layer and includes pulp fibers, and (iii) a second hydrophilic fiber containing layer which is adjacent to the pulp fiber containing layer and includes second hydrophilic fibers with an average fiber length of 25 mm to 75 mm, and

a portion of the pulp fibers in the pulp fiber containing layer penetrates the first hydrophilic fiber containing layer and is in contact with the absorbent core.

In the absorbent body of aspect 1, when liquid such as body fluid, etc., is supplied to the core wrapping sheet (the first liquid supply), the liquid diffuses in the surface of the liquid permeable sheet with the liquid supply point as the center, and the liquid also permeates in the thickness direction of the absorbent body with the liquid supply point as the center. When the liquid permeates in the thickness direction of the absorbent body and reaches the core wrapping sheet, the liquid diffuses in the plane direction of the core wrapping sheet by "the diffusing function" of the core wrapping sheet. The above mentioned "diffusing function" is achieved mainly by the core wrapping sheet having a sandwiched configuration in which the pulp fiber containing layer is sandwiched by the first hydrophilic fiber containing layer and the second hydrophilic fiber containing layer.

To be more specific, by the capillary force caused by the first hydrophilic fibers included in the first hydrophilic fiber containing layer and the capillary force caused by the second hydrophilic fibers included in the second hydrophilic fiber containing layer, the liquid diffuses in the plane direction of the absorbent body in these layers. The liquid which has diffused in the inside of each of the first hydrophilic fiber containing layer and the second hydrophilic fiber containing layer is delivered to the pulp fiber containing layer by the high hydrophilicity of the adjacent pulp fiber containing layer, and repeats a diffusion - delivery cycle of diffusion of the liquid inside the first hydrophilic fiber containing layer and the second hydrophilic fiber containing layer and the delivery of the liquid to the pulp fiber containing layer, whereby the liquid diffuses in the plane direction inside the core wrapping sheet, and also the liquid is temporarily stored inside the pulp fiber containing layer. Subsequently, the liquid which has been temporarily stored inside the pulp fiber containing layer is transferred to the absorbent core, through the pulp fibers which penetrate the first hydrophilic fiber containing layer and are connected to the absorbent core, so as to be absorbed by the polymer absorbing agents inside the absorbent core. Accordingly, the core wrapping sheet can prepare for the next liquid supply.

When the liquid is supplied to the core wrapping sheet again (the second liquid supply), the liquid diffuses in the surface of the liquid permeable sheet with the liquid supply point as the center, and the liquid also permeates in the thickness direction of the absorbent body with the liquid supply point as the center. When the liquid permeates in the thickness direction of the absorbent body and reaches the core wrapping sheet, the liquid diffuses quickly to a liquid permeation region of the core wrapping sheet which is the diffused range at the first liquid supply. This is because the affinity to liquid of the first hydrophilic fibers in the first hydrophilic fiber containing layer and of the second hydrophilic fibers in the second hydrophilic fiber has increased.

Subsequently, in the core wrapping sheet, the liquid repeats the diffusion - delivery cycle beyond the diffused range at the first liquid supply, and diffuses to a position farther from the liquid supply point compared to the first liquid supply, and also the liquid is temporarily stored inside the pulp fiber containing layer. The liquid which has been temporarily stored inside the pulp fiber containing layer is transferred to the absorbent core, through the pulp fibers which penetrate the first hydrophilic fiber containing layer and are connected to the absorbent core, so as to be absorbed by the polymer absorbing agents which are present in a wider range inside the absorbent core compared to the first liquid supply. Accordingly, the core wrapping sheet can prepare for the next liquid supply.

When the liquid is supplied to the core wrapping sheet again (the third liquid supply), the liquid diffuses to a position farther from the liquid supply point compared to the second liquid supply, and also is absorbed by the polymer absorbing agents which are present in a wider range inside the absorbent core compared to the second liquid supply, in the same manner.

Accordingly, the absorbent body described in aspect 1 is excellent in diffusing liquid in the plane direction and in transferring the liquid in the thickness direction, when the liquid is repeatedly absorbed.

[Aspect 2]

The absorbent body according to aspect 1, wherein the absorbent core includes the polymer absorbing agent in a surface of the absorbent core, and

the portion of the pulp fibers in the pulp fiber containing layer penetrates the first hydrophilic fiber containing layer and is in contact with the polymer absorbing agent in the surface of the absorbent core.

In the absorbent body described in aspect 2, the portion of the pulp fibers in the pulp fiber containing layer penetrates the first hydrophilic fiber containing layer and is in contact with the polymer absorbing agent in the surface of the absorbent core. Accordingly, the liquid which has been temporarily stored in the pulp fiber containing layer is absorbed by the polymer absorbing agents through the pulp fibers which penetrate the first hydrophilic fiber containing layer in response to the requirement of the polymer absorbing agents, whereby the absorbent body is excellent in absorbing liquid.

[Aspect 3]

The absorbent body according to aspect 2, wherein the first hydrophilic fiber containing layer is fixed to the polymer absorbing agent in the surface of the absorbent core through an adhesion layer.

In the absorbent body described in aspect 3, the polymer absorbing agent which is present in the surface of the absorbent core is fixed to the first hydrophilic fiber containing layer of the core wrapping sheet through the adhesion layer. Accordingly, even when the absorbent body is deformed when being worn, it is easy for the connection between the polymer absorbing agent and the pulp fiber containing layer through the pulp fibers to be retained. As a result, even when the wearer moves intensely, the liquid which has been temporarily stored in the pulp fiber containing layer is directly absorbed by the polymer absorbing agents through the pulp fibers which penetrate the first hydrophilic fiber containing layer in response to the requirement of the polymer absorbing agents, whereby the absorbent body is excellent in absorbing liquid.

[Aspect 4]

The absorbent body according to any one of aspects 1 to 3, wherein

the absorbent body includes the core wrapping sheet at least on a skin side surface of the absorbent core, and

in the core wrapping sheet which is disposed in the skin side surface of the absorbent core, the portion of the pulp fibers in the pulp fiber containing layer penetrates the second hydrophilic fiber containing layer and reaches a surface of the second hydrophilic fiber containing layer on an opposite side of the pulp fiber containing layer.

In the absorbent body described in aspect 4, the core wrapping sheet is provided on the skin side surface of the absorbent core, and in the core wrapping sheet which is disposed in the skin side surface of the absorbent body, the portion of the pulp fibers in the pulp fiber containing layer penetrates the second hydrophilic fiber containing layer and reaches the surface of the second hydrophilic fiber containing layer on an opposite side of the pulp fiber containing layer. Accordingly, while quickly drawing the liquid which is present in the layer adjacent to the second hydrophilic fiber containing layer (for example, the liquid permeable sheet of the absorbent article) into the second hydrophilic fiber containing layer, the liquid can be diffused in the plane direction of the second hydrophilic fiber containing layer and further, can be quickly delivered to the pulp fiber containing layer, whereby the absorbent body is excellent in diffusing liquid in the plane direction and in transferring the liquid in the thickness direction, and is also excellent in the absorption speed of the liquid.

[Aspect 5]

The absorbent body according to any one of aspects 1 to 4, wherein each of the first hydrophilic fibers and the second hydrophilic fibers are cellulose based fibers.

In the absorbent body described in aspect 5, the first hydrophilic fibers and the second hydrophilic fibers are cellulose based fibers. Accordingly, each of the first hydrophilic fiber containing layer and the second hydrophilic fiber containing layer is excellent in diffusing the liquid in the plane direction and can deliver the liquid to the pulp fibers in the pulp fiber containing layer. Accordingly, the absorbent body is excellent in the above described diffusion - delivery cycle, and is excellent in absorbing liquid.

[Aspect 6]

The absorbent body according to any one of aspects 1 to 5, wherein the pulp fibers in the pulp fiber containing layer have an average fiber length of 1 mm to 10 mm.

In the absorbent body described in aspect 6, since the average fiber length of the pulp fibers is 1 mm to 10 mm, it is easier for the pulp fibers to penetrate the second hydrophilic fiber containing layer and for the liquid which has been temporarily stored in the pulp fiber containing layer to be transferred to the absorbent core through the second hydrophilic fiber containing layer. As a result, the absorbent body is excellent in absorbing liquid.

[Aspect 7]

The absorbent body according to any one of aspects 1 to 6, wherein the core wrapping sheet has a basis weight of 30 g / m$^2$ to 100 g / m$^2$.

In the absorbent body described in aspect 7, the core wrapping sheet has a predetermined basis weight, whereby the core wrapping sheet is excellent in diffusing liquid in the plane direction in the first hydrophilic fiber containing layer and the second hydrophilic fiber containing layer, and in temporarily storing liquid in the pulp fiber containing layer, and thus the absorbent body of the present disclosure is excellent in absorbing liquid.

[Aspect 8]

The absorbent body according to any one of aspects 1 to 7, wherein the core wrapping sheet has a density of 0.05 g / cm$^3$ to 0.20 g / cm$^3$.

In the absorbent body described in aspect 8, the core wrapping sheet has a predetermined density, whereby has a moderate capillary force, and is excellent in drawing liquid to the core wrapping sheet and in delivering liquid from the core wrapping sheet to the absorbent core. As a result, the absorbent body and the absorbent article which includes the absorbent body are excellent in rewetting property.

[Aspect 9]

The absorbent body according to any one of aspects 1 to 8, wherein the core wrapping sheet has a diffusion length of 130 mm or more, and a transfer amount of 31.0 g or more, in a diffusion - transfer test.

In the absorbent body described in aspect 9, the core wrapping sheet has a predetermined diffusion length and transfer amount, whereby the absorbent body is excellent in diffusing liquid in the plane direction and in transferring the liquid in the thickness direction, and is also excellent in the absorption speed of the liquid.

[Aspect 10]

The absorbent body according to any one of aspects 1 to 9, wherein the core wrapping sheet is adjacently disposed in both surfaces of a skin side surface and a non-skin side surface of the absorbent core.

In the absorbent body described in aspect 10, the core wrapping sheet is adjacently disposed in both surfaces of the skin side surface and the non-skin side surface of the absorbent core. Accordingly, even when a large amount of liquid is supplied at once to the absorbent body, the cycle of diffusing and delivering liquid in the core wrapping sheet can be repeated in both of the skin side surface and the non-skin side surface of the absorbent core, and as a result, the absorbent body is excellent in absorbing liquid.

[Aspect 11]

The absorbent body according to aspect 10, wherein
the absorbent core includes the polymer absorbing agents in the both surfaces of the skin side surface and the non-skin side surface of the absorbent core, and
the pulp fibers in the pulp fiber containing layer of the core wrapping sheet disposed in the skin side surface and the non-skin side surface are in contact with the polymer absorbing agents in the skin side surface and the non-skin side surface, respectively, of the absorbent core.

In the absorbent body described in aspect 11, the pulp fibers in the pulp fiber containing layer of the core wrapping sheet disposed in the skin side surface and the non-skin side surface are in contact with the polymer absorbing agents in the skin side surface and the non-skin side surface, respectively, of the absorbent core. Accordingly, even when a large amount of liquid is supplied at once to the absorbent body, the cycle of diffusing and delivering liquid in the core wrapping sheet can be repeated in both of the skin side surface and the non-skin side surface of the absorbent core, and as a result, the absorbent body is excellent in absorbing liquid.

[Aspect 12]

The absorbent body according to aspect 11, wherein the absorbent core includes a hydrophilic fabric between the polymer absorbing agent which is present in the skin side surface and the polymer absorbing agent which is present in the non-skin side surface.

The absorbent body described in aspect 12 includes a fabric of hydrophilic fibers between the polymer absorbing agent which is present in the skin side surface and the polymer absorbing agent which is present in the non-skin side surface. Accordingly, when the polymer absorbing agents absorb the liquid, it is difficult for the blocking of the polymer absorbing agents to occur, and as a result, the absorbent body is excellent in absorbing liquid.

Hereinbelow, the absorbent body of the present disclosure is explained in detail.

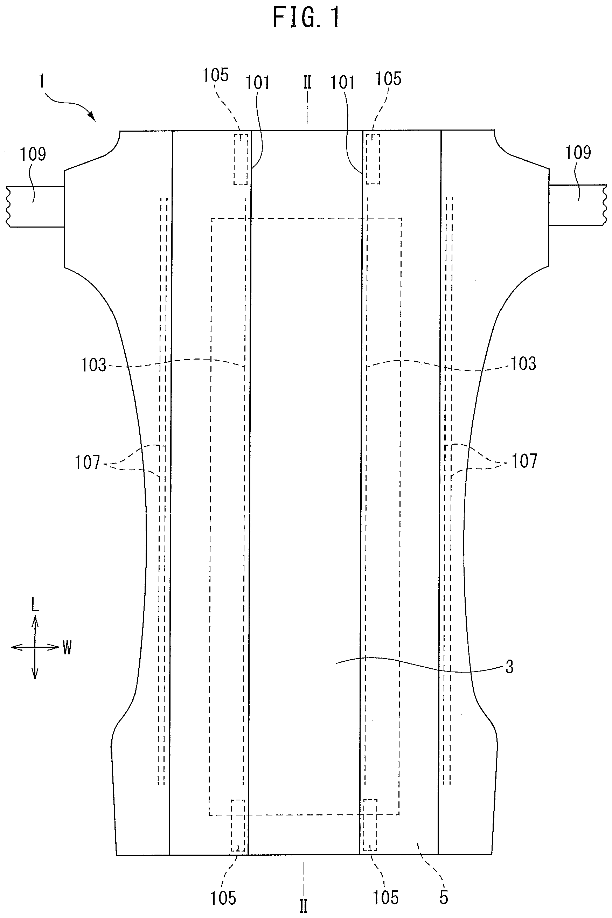

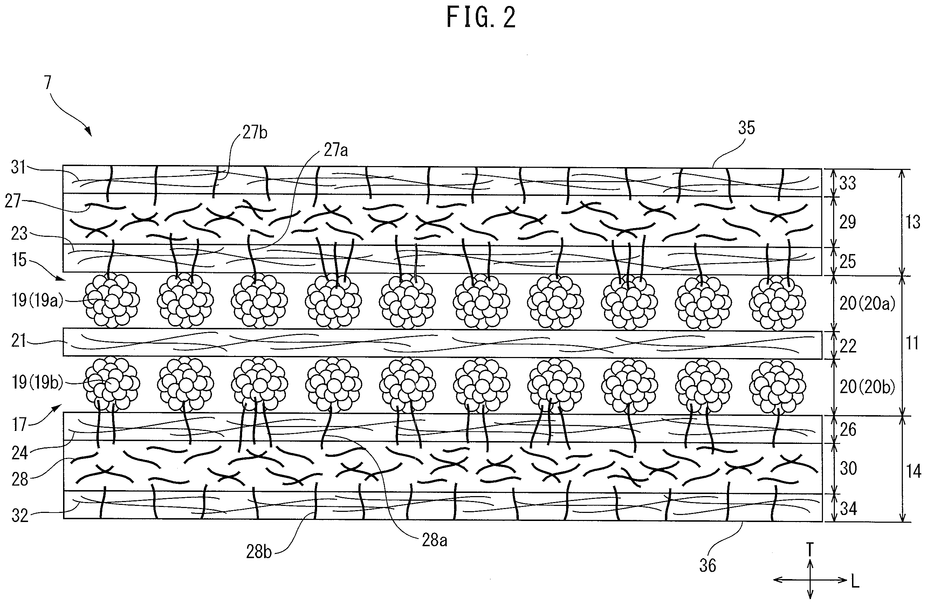

FIGS. 1 and 2 are views for explaining an absorbent article 1, to be more specific, a tape type disposable diaper, which includes an absorbent body 7 according to one embodiment (the first embodiment) of the present disclosure. To be more specific, FIG. 1 is an expanded view of the absorbent article 1 which includes the absorbent body 7 according to the first embodiment, and FIG. 2 is an end view of the absorbent body 7 at a II - II end surface of FIG. 1.

The absorbent article 1 shown in FIG. 1 has a longitudinal direction L and a width direction W, and includes a liquid permeable sheet 3, a liquid impermeable sheet 5, and the absorbent body 7 between the liquid permeable sheet 3 and the liquid impermeable sheet 5. The absorbent article 1 further includes a pair of leakage prevention barriers 101 each of which including an elastic member 103, fixed portions 105 to fix the leakage prevention barriers 101 to the liquid permeable sheet 3, elastic members 107, tape fasteners 109, etc., however, since these configurations are known in the technical field, the description thereof is omitted.

As shown in FIG. 1, the absorbent body 7 according to the first embodiment, in an expanded state, has a substantially rectangular shape, and has the same longitudinal direction L and the width direction W, as the absorbent article 1. As shown in FIG. 2, the absorbent body 7 includes an absorbent core 11 and core wrapping sheets 13, 14. To be more specific, the absorbent body 7 includes the absorbent core 11, the core wrapping sheet 13 which is disposed adjacent to the skin side surface 15 of the absorbent core 11, and the core wrapping sheet 14 which is disposed adjacent to the non-skin side surface 17 of the absorbent core 11.

The absorbent core 11 includes polymer absorbing agents 19, and to be more specific, the absorbent core 11 includes a polymer absorbing agent layer 20a composed of polymer absorbing agents 19a disposed in the skin side surface 15 of the absorbent core 11, a polymer absorbing agent layer 20b composed of polymer absorbing agents 19b disposed in the non-skin side surface 17 of the absorbent core 11, and a layer of a hydrophilic fabric 21 (hydrophilic fabric layer 22) disposed between the polymer absorbing agent layer 20a and the polymer absorbing agent layer 20b.

The core wrapping sheet 13 includes (i) a first hydrophilic fiber containing layer 25 which is adjacent to the absorbent core 11 and is composed of first hydrophilic fibers 23, (ii) a pulp fiber containing layer 29 which is adjacent to the first hydrophilic fiber containing layer 25 and is composed of pulp fibers 27, and (iii) a second hydrophilic fiber containing layer 33 which is adjacent to the pulp fiber containing layer 29 and is composed of second hydrophilic fibers 31.

In the same manner, the core wrapping sheet 14 includes (i) a first hydrophilic fiber containing layer 26 which is adjacent to the absorbent core 11 and is composed of first hydrophilic fibers 24, (ii) a pulp fiber containing layer 30 which is adjacent to the first hydrophilic fiber containing layer 26 and is composed of pulp fibers 28, and (iii) a second hydrophilic fiber containing layer 34 which is adjacent to the pulp fiber containing layer 30 and is composed of second hydrophilic fibers 32.

Each of the first hydrophilic fibers 23, the first hydrophilic fibers 24, the second hydrophilic fibers 31, and the second hydrophilic fibers 32 have an average fiber length of 25 mm to 75 mm.

In the core wrapping sheet 13, pulp fibers 27a which are a portion of the pulp fibers 27 in the pulp fiber containing layer 29 penetrate the first hydrophilic fiber containing layer 25, and are in direct contact with the absorbent core 11, and to be more specific, with the polymer absorbing agents 19a of the polymer absorbing agent layer 20a disposed in the skin side surface 15 of the absorbent core 11. Further, the first hydrophilic fiber containing layer 25 is fixed to the polymer absorbing agents 19a of the polymer absorbing agent layer 20a through an adhesion layer (which is not shown).

In the same manner, in core wrapping sheet 14, pulp fibers 28a which are a portion of the pulp fibers 28 in the pulp fiber containing layer 30 penetrate the first hydrophilic fiber containing layer 26, and are in direct contact with the absorbent core 11, and to be more specific, with the polymer absorbing agents 19b of the polymer absorbing agent layer 20b disposed in the non-skin side surface 17 of the absorbent core 11. Further, the first hydrophilic fiber containing layer 26 is fixed to the polymer absorbing agents 19b of the polymer absorbing agent layer 20b through an adhesion layer (which is not shown).

As shown in FIG. 2, in the core wrapping sheet 13, the pulp fibers 27b which are the portion of the pulp fibers 27 in the pulp fiber containing layer 29 penetrate the second hydrophilic fiber containing layer 33 and are in direct contact with the surface 35 of the second hydrophilic fiber containing layer 33 on an opposite side of the pulp fiber containing layer 29, and to be more specific, with the liquid permeable sheet 3.

Further, as shown in FIG. 2, in the core wrapping sheet 14, the pulp fibers 28b which are the portion of the pulp fibers 28 in the pulp fiber containing layer 30 penetrate the second hydrophilic fiber containing layer 34 and are in direct contact with the surface 36 of the second hydrophilic fiber containing layer 34 on an opposite side of the pulp fiber containing layer 30, and to be more specific, with the liquid permeable sheet (which is not shown).

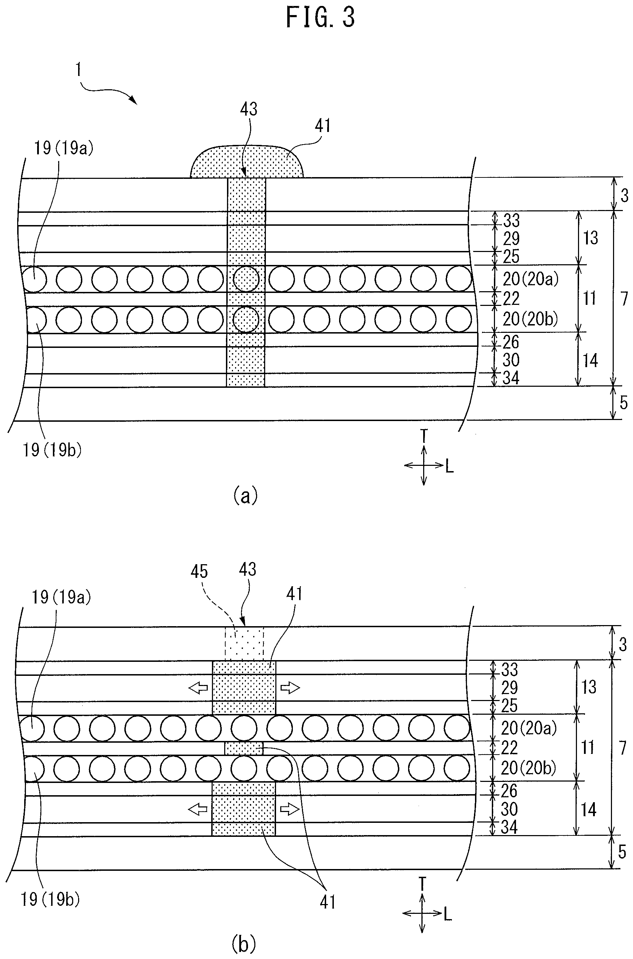

FIGS. 3 to 7 are views for explaining the excellence in diffusing liquid in the plane direction and in transferring the liquid in the thickness direction, when the absorbent body 7 according to the first embodiment repeatedly absorbs liquid. FIGS. 3 to 7 are end views which correspond to the II - II end surface of FIG. 1, and for illustration purposes, the first hydrophilic fibers, the second hydrophilic fibers, and the pulp fibers are omitted, and the polymer absorbing agents 19 are expressed as circles.

<First liquid supply>

In the first liquid supply, as shown in FIG. 3(a), when liquid 41 such as body fluid, etc., is supplied to the liquid permeable sheet 3 of the absorbent article 1, the liquid 41 diffuses in the surface of the liquid permeable sheet 3 with the liquid supply point 43 as the center in the longitudinal direction L (and in the width direction) of the absorbent article 1, and the liquid 41 also permeates in the thickness direction T of the absorbent body 7 with the liquid supply point 43 as the center.

As shown in FIG. 3(b), the liquid 41 permeates in the thickness direction T of the absorbent body 7, and the liquid 41 which is transferred from the liquid permeable sheet 3 to the core wrapping sheet 13 and the core wrapping sheet 14 diffuses in the plane direction of the absorbent article 1 (the absorbent body 7), and to be more specific, in the longitudinal direction L and the width direction (which is not shown) of the absorbent article 1 (the absorbent body 7), in each of the core wrapping sheet 13 and the core wrapping sheet 14, by the diffusing function of each of the core wrapping sheet 13 and the core wrapping sheet 14. In the liquid permeable sheet 3, a liquid permeation region 45 is formed at the portion in which the liquid 41 has permeated. The liquid permeation region 45 has once been in contact with the liquid 41, whereby is the region which has high affinity to the liquid 41.

The above mentioned diffusing function is achieved by the core wrapping sheet 13 having a sandwiched configuration in which the pulp fiber containing layer 29 is sandwiched by the first hydrophilic fiber containing layer 25 and the second hydrophilic fiber containing layer 33.

To be more specific, by the capillary force caused by the first hydrophilic fibers (which are not shown) included in the first hydrophilic fiber containing layer 25 and the capillary force caused by the second hydrophilic fibers (which are not shown) included in the second hydrophilic fiber containing layer 33, the liquid 41 diffuses in the plane direction of the absorbent body 7 inside the first hydrophilic fiber containing layer 25 and the second hydrophilic fiber containing layer 33. Incidentally, since the operations in the core wrapping sheet 14 work in the same manner, the description thereof is omitted.

The liquid 41 which has diffused in the plane direction of the absorbent body 7, in each of the first hydrophilic fiber containing layer 25 and the second hydrophilic fiber containing layer 33 is transferred to the pulp fiber containing layer 29 by the high hydrophilicity of the pulp fibers (which are not shown) in the adjacent pulp fiber containing layer 29. The liquid 41 is transferred to the pulp fiber containing layer 29, whereby in each of the first hydrophilic fiber containing layer 25 and the second hydrophilic fiber containing layer 33, a liquid permeation region (which is not shown) is formed in the region in which the liquid 41 is retained, and also, rooms for accepting the new liquid 41 are to be made in each of the first hydrophilic fiber containing layer 25 and the second hydrophilic fiber containing layer 33.

In each of the first hydrophilic fiber containing layer 25 and the second hydrophilic fiber containing layer 33, since the liquid permeation region (which is not shown) has once been in contact with the liquid 41, and is the region which has high affinity to the liquid 41, when the new liquid 41 which remains in the liquid permeable sheet 3 reaches the liquid permeation region (which is not shown) in the first hydrophilic fiber containing layer 25 and the second hydrophilic fiber containing layer 33, the new liquid 41 diffuses farther in the plane direction of the absorbent body 7 beyond the liquid permeation region (which is not shown), in each of the first hydrophilic fiber containing layer 25 and the second hydrophilic fiber containing layer 33.

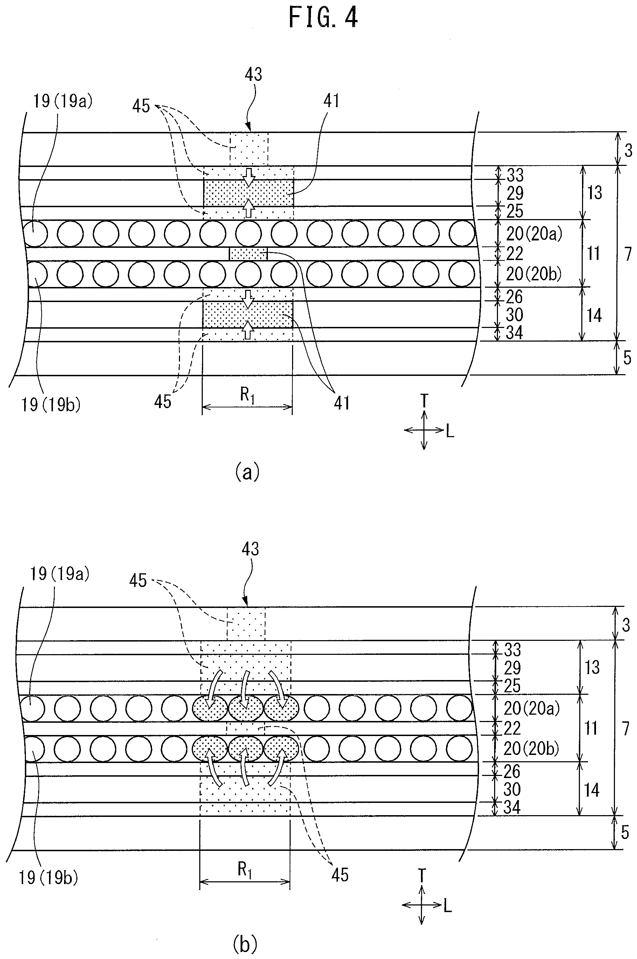

The liquid 41 repeats the diffusion - delivery cycle of diffusion of the liquid inside the first hydrophilic fiber containing layer 25 and the second hydrophilic fiber containing layer 33 and the delivery of the liquid to the pulp fiber containing layer 29, until the supplied liquid 41 runs out or until the upper limit at which the liquid 41 can be stored in the pulp fiber containing layer 29 is reached. As a result, the liquid 41, as shown in FIG. 4(a), diffuses over the region : R1 in the plane direction of the absorbent body 7 inside the core wrapping sheet 13, and also is temporarily stored in the pulp fiber containing layer 29. Incidentally, since the operations in the core wrapping sheet 14 work in the same manner, the description thereof is omitted.

As shown in FIG. 4(b), in the region : R1, the liquid 41 which has been temporarily stored inside the pulp fiber containing layer 29 is transferred to the absorbent core 11, through the pulp fibers (which are not shown) which penetrate the first hydrophilic fiber containing layer 25 and are connected to the absorbent core 11, so as to be absorbed by the polymer absorbing agents 19a of the absorbent core 11. Referring to FIG. 2, since the pulp fibers 27a which are the portion of the pulp fibers 27 in the pulp fiber containing layer 29 penetrate the first hydrophilic fiber containing layer 25, and are in direct contact with the absorbent core 11, and to be more specific, with the polymer absorbing agents 19a of the polymer absorbing agent layer 20a disposed in the skin side surface 15 of the absorbent core 11, when the polymer absorbing agents 19a absorb the liquid 41, the liquid 41 which has been temporarily stored inside the pulp fiber containing layer 29 is quickly supplied to the polymer absorbing agents 19a through the pulp fibers 27a. As a result, in the region : R1, the liquid 41 which has been temporarily stored inside the pulp fiber containing layer 29 is quickly absorbed by the polymer absorbing agents 19a, and the core wrapping sheet 13 can prepare for the next supply of the liquid 41. Incidentally, since the operations in the core wrapping sheet 14 work in the same manner, the description thereof is omitted.

<Second liquid supply>

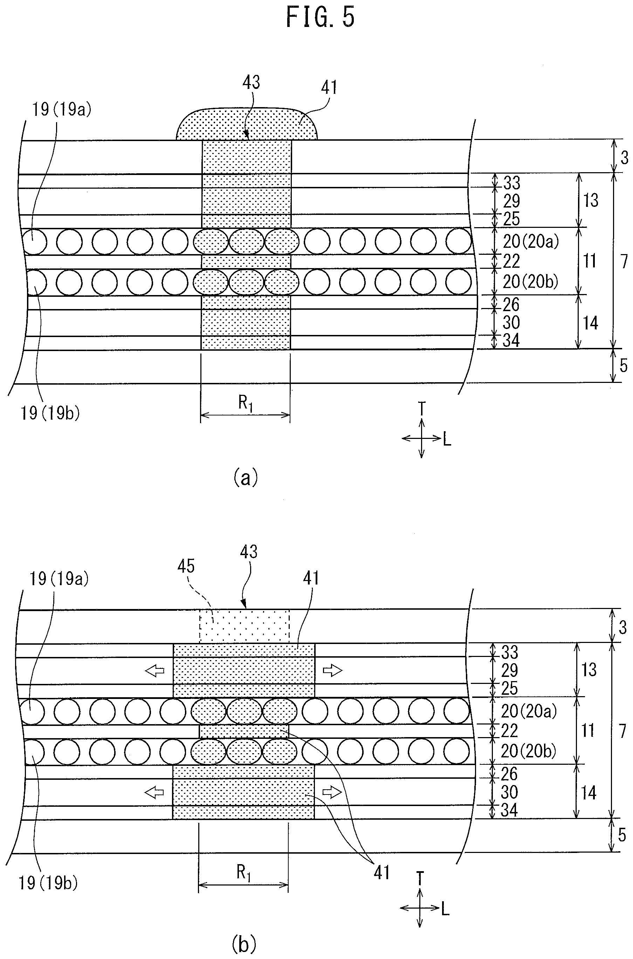

In the second liquid supply, as shown in FIG. 5(a), when liquid 41 such as body fluid, etc., is supplied to the liquid permeable sheet 3 of the absorbent article 1, the liquid 41 diffuses in the surface of the liquid permeable sheet 3 with the supply point 43 of the liquid 41 as the center in the longitudinal direction L of the absorbent article 1, and the liquid 41 also permeates in the thickness direction T of the absorbent body 7 with the supply point 43 as the center. When the liquid 41 permeates in the thickness direction T of the absorbent body 7 and reaches the core wrapping sheet 13 and the core wrapping sheet 14, the liquid 41 diffuses quickly to the region : R1 which is the region in the core wrapping sheet 13 and the core wrapping sheet 14 in which the liquid 41 has diffused at the first liquid supply (the liquid permeation region).

As shown in FIG. 5(b), the liquid 41 permeates in the thickness direction T of the absorbent body 7, and the liquid 41 which is transferred from the liquid permeable sheet 3 to the core wrapping sheet 13 and the core wrapping sheet 14 diffuses in the plane direction of the absorbent body 7 in each of the core wrapping sheet 13 and the core wrapping sheet 14, beyond the region : R1 in which the liquid 41 has diffused in the first liquid supply, by the diffusing function of each of the core wrapping sheet 13 and the core wrapping sheet 14. Incidentally, the description of the diffusing function is given above.

Incidentally, in the liquid permeable sheet 3, the liquid permeation region 45 which is wider than that of the first liquid supply is formed at the portion in which the liquid 41 has permeated. Incidentally, since the operations in the core wrapping sheet 14 work in the same manner, the description thereof is omitted.

The liquid 41 which has diffused in the plane direction of the absorbent body 7, in each of the first hydrophilic fiber containing layer 25 and the second hydrophilic fiber containing layer 33 is delivered to the pulp fiber containing layer 29 by the high hydrophilicity of the pulp fibers (which are not shown) in the adjacent pulp fiber containing layer 29. The liquid 41 is delivered to the pulp fiber containing layer 29, whereby in each of the first hydrophilic fiber containing layer 25 and the second hydrophilic fiber containing layer 33, the liquid permeation region 45 is formed in the region in which the liquid 41 is retained, and also, rooms for accepting the new liquid 41 are to be made in each of the first hydrophilic fiber containing layer 25 and the second hydrophilic fiber containing layer 33. Incidentally, since the operations in the core wrapping sheet 14 work in the same manner, the description thereof is omitted.

In each of the first hydrophilic fiber containing layer 25 and the second hydrophilic fiber containing layer 33, since the liquid permeation region (which is not shown) has once been in contact with the liquid 41, and is the region which has high affinity to the liquid 41, when the new liquid 41 which remains in the liquid permeable sheet 3 reaches the liquid permeation region (which is not shown) in the first hydrophilic fiber containing layer 25 and the second hydrophilic fiber containing layer 33, the new liquid 41 diffuses farther in the plane direction of the absorbent body 7 beyond the liquid permeation region (which is not shown), in each of the first hydrophilic fiber containing layer 25 and the second hydrophilic fiber containing layer 33.

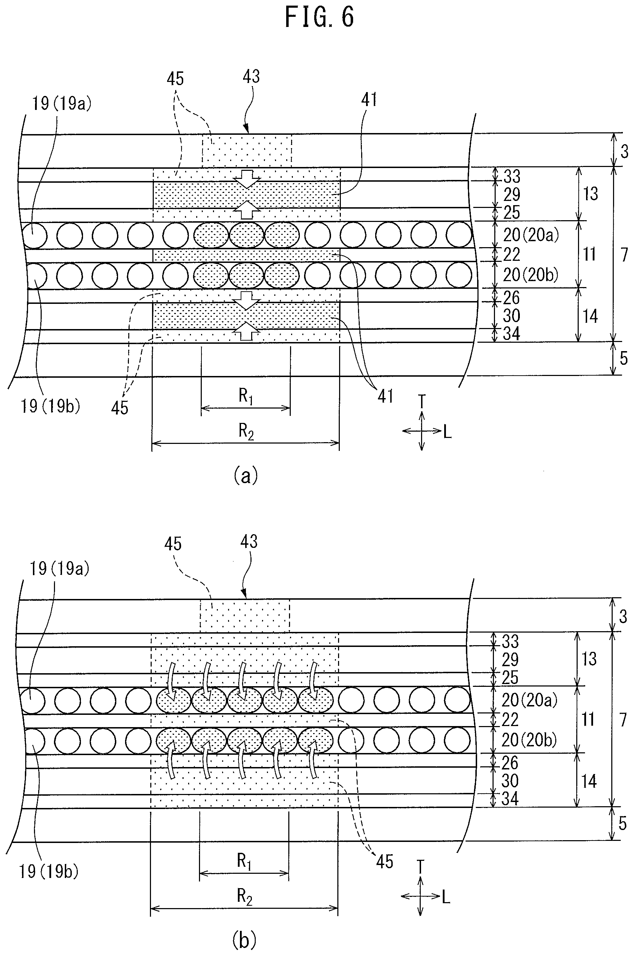

The liquid 41 repeats the diffusion - delivery cycle of diffusion of the liquid inside the first hydrophilic fiber containing layer 25 and the second hydrophilic fiber containing layer 33 and the delivery of the liquid to the pulp fiber containing layer 29, until the supplied liquid 41 runs out or until the upper limit at which the liquid 41 can be stored in the pulp fiber containing layer 29 is reached. As a result, the liquid 41, as shown in FIG. 6(b), diffuses to the region : R2 in the plane direction of the absorbent body 7 inside the core wrapping sheet 13, and also is temporarily stored in the pulp fiber containing layer 29. Incidentally, since the operations in the core wrapping sheet 14 work in the same manner, the description thereof is omitted.

As shown in FIG. 6(b), in the region : R2, the liquid 41 which has been temporarily stored inside the pulp fiber containing layer 29 is transferred to the absorbent core 11, through the pulp fibers (which are not shown) which penetrate the first hydrophilic fiber containing layer 25 and are connected to the absorbent core 11, so as to be absorbed by the polymer absorbing agents 19a of the absorbent core 11. The absorption mechanism works in the same manner as that explained in FIG. 4(b) in relation to the first liquid supply, however, is different in that the liquid 41 is absorbed by the polymer absorbing agents 19a in the region : R2 which is wider than the region : R1 in which the liquid 41 has diffused in the first liquid supply.

As a result, in the region : R2, the liquid 41 which has been temporarily stored inside the pulp fiber containing layer 29 is quickly absorbed by the polymer absorbing agents 19a, and the core wrapping sheet 13 can prepare for the next supply of the liquid 41. Incidentally, since the operations in the core wrapping sheet 14 work in the same manner, the description thereof is omitted.

<Third liquid supply>

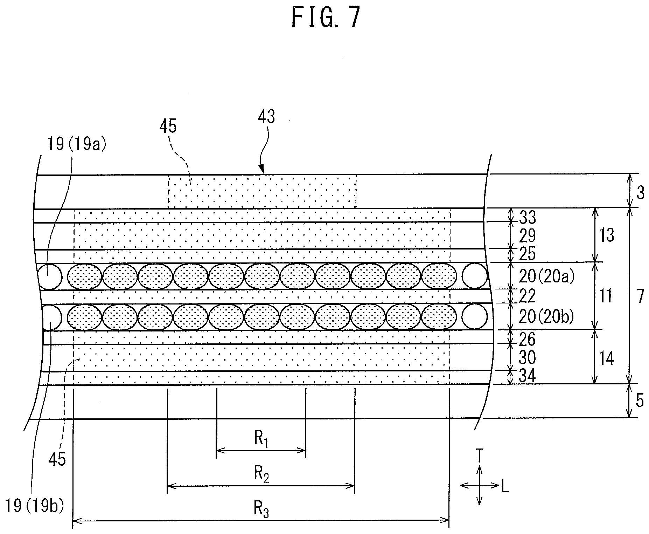

Also in the third liquid supply, the absorbent article 1 repeats the absorption in the same manner as that in the first liquid supply and in the second liquid supply, and as a result, as shown in FIG. 7, the liquid 41 is absorbed by the polymer absorbing agents 19a in the region : R3 which is wider than the region : R2 in which the liquid 41 has diffused in the second liquid supply. Incidentally, since the operations in the core wrapping sheet 14 work in the same manner, the description thereof is omitted.

As described above, the absorbent body 7 is excellent in diffusing the liquid 41 in the plane direction and in transferring the liquid 41 in the thickness direction, when the liquid 41 is repeatedly absorbed.

In the first embodiment, the core wrapping sheet 13 is composed of the first hydrophilic fiber containing layer 25, the second hydrophilic fiber containing layer 33, and the pulp fiber containing layer 29 (the pulp fiber containing layer 30) therebetween, however, the absorbent body of the present disclosure may include additional layers, to the extent that the effect of the present disclosure is achieved.

In the first embodiment, the first hydrophilic fiber containing layer 25 of the core wrapping sheet 13 is composed of the first hydrophilic fibers 23, however, in the absorbent body of the present disclosure, the first hydrophilic fiber containing layer which configures the core wrapping sheet includes the first hydrophilic fibers by preferably 50 mass % or more, by more preferably 70 mass % or more, by even more preferably 90 mass % or more, and by still even more preferably 100 mass %. This is from the viewpoint of the effect of the present disclosure.

Further, in the first embodiment, the second hydrophilic fiber containing layer 33 of the core wrapping sheet 13 is composed of the second hydrophilic fibers 31, however, in the absorbent body of the present disclosure, the second hydrophilic fiber containing layer which configures the core wrapping sheet includes the second hydrophilic fibers by preferably 50 mass % or more, by more preferably 70 mass % or more, by even more preferably 90 mass % or more, and by still even more preferably 100 mass %. This is from the viewpoint of the effect of the present disclosure.

Each of the first hydrophilic fibers and the second hydrophilic fibers preferably have an average fiber length which is longer than an average fiber length of the pulp fibers which configure the pulp fiber containing layer, and have an average fiber length preferably of 25 mm to 75 mm, more preferably of 30 mm to 65 mm, and even more preferably of 35 mm to 55 mm. This is from the viewpoint of the effect of the present disclosure, especially, the diffusing property in the plane direction in the first hydrophilic fiber containing layer and the second hydrophilic fiber containing layer.

Each of the first hydrophilic fibers and the second hydrophilic fibers are not particularly limited as long as each of the first hydrophilic fibers and the second hydrophilic fibers have hydrophilicity, and from the viewpoint of being capable of retaining the hydrophilicity thereof even when liquid is absorbed a plurality of times, the material itself is preferably hydrophilic. As each of the first hydrophilic fibers and the second hydrophilic fibers, for example, synthetic fibers, natural fibers, semi-synthetic fibers, etc., may be mentioned.

As the above mentioned synthetic fibers, for example, fibers configured by polyolefin-based polymers, such as polyethylene or polypropylene; polyester-based polymers, such as, terephthalate-based polymers, for example, polyethylene terephthalate (PET), polybutylene terephthalate, polypentylene terephthalate; polyamide-based polymers, such as nylon 6 or nylon 6, 6; acrylic-based polymers; polyacrylonitrile-based polymers; or modified products of these materials, or the combination of these materials, etc., may be mentioned.

As the above mentioned natural fibers, regenerated cellulose fibers, purified cellulose fibers, etc., may be mentioned.

As the regenerated cellulose fibers, fibers of rayon, for example, viscose rayon obtained from viscose, polynosic and modal, copper ammonium rayon obtained from copper ammonium salt solution of cellulose (which is also referred to as "cupra"), etc., may be mentioned.

As the purified cellulose fibers, lyocell, and to be more specific, fibers made of pulp being dissolved in an aqueous solution of N-methylmorpholine N-oxide to prepare a spinning solution (dope), and being extruded into a dilute solution of N-methylmorpholine N-oxide. The above mentioned purified cellulose fibers are commercially available for example as Tencel (trademark).

As the above mentioned semi-synthetic fibers, fibers of semi-synthetic cellulose, for example, acetate fibers such as triacetate and diacetate, etc., may be mentioned.

In the first embodiment, the pulp fiber containing layer 29 of the core wrapping sheet 13 is composed of the pulp fibers 27, however, in the absorbent body of the present disclosure, the pulp fiber containing layer which configures the core wrapping sheet includes the pulp fibers by preferably 50 mass % or more, by more preferably 70 mass % or more, by even more preferably 90 mass % or more, and by still even more preferably 100 mass %. This is from the viewpoint of the effect of the present disclosure.

The average fiber length of the pulp fibers are not particularly limited, however, is preferably longer than the thickness of the first hydrophilic fiber containing layer. This is because it is possible for a single pulp fiber to penetrate the first hydrophilic fiber containing layer. Further, in an embodiment in which pulp fibers penetrate the second hydrophilic fiber containing layer, the average fiber length of the pulp fibers is preferably longer than the thickness of the second hydrophilic fiber containing layer. This is because it is possible for a single pulp fiber to penetrate the second hydrophilic fiber containing layer.

The pulp fibers have an average fiber length of preferably 10 mm or shorter, and more preferably 6 mm or shorter. This is from the viewpoint of transferring the absorbed liquid not in the plane direction of the absorbent body but mainly in the thickness direction of the absorbent body. The pulp fibers have an average fiber length of preferably 1 mm or longer, and more preferably 2 mm or longer. This is from the viewpoint of handling the pulp fibers.

The above mentioned pulp fibers include pulp fibers which are known in the technical field, and for example, wood pulp and non-wood pulp may be mentioned. As the wood pulp, for example, coniferous pulp and hardwood pulp may be mentioned. As the non-wood pulp, for example, straw pulp, bagasse pulp, reed pulp, kenaf pulp, mulberry pulp, bamboo pulp, hemp pulp, cotton pulp (for example, cotton linter), etc., may be mentioned.

In the core wrapping sheet 13 of the absorbent body 7 according to the first embodiment, as shown in FIG. 2, a single fiber of the pulp fibers 27a which are a portion of the pulp fibers 27 in the pulp fiber containing layer 29 penetrates the first hydrophilic fiber containing layer 25, and is in direct contact with the absorbent core 11, and to be more specific, with the polymer absorbing agents 19a of the polymer absorbing agent layer 20a disposed in the skin side surface 15 of the absorbent core 11. That is, one end portion of the single fiber of the pulp fibers 27a is inside the pulp fiber containing layer 29, and the other end portion thereof is in direct contact with the polymer absorbing agents 19a of the polymer absorbing agent layer 20a.

In the core wrapping sheet of the absorbent body of the present disclosure, the pulp fibers which penetrate the first hydrophilic fiber containing layer may be a blob of the pulp fibers, that is, a plurality of pulp fibers may integrally penetrate the first hydrophilic fiber containing layer so as to be in contact with the absorbent core, or as in the first embodiment, a single pulp fiber may penetrate the first hydrophilic fiber containing layer so as to be in contact with the absorbent core. In a case in which the pulp fibers penetrate the first hydrophilic fiber containing layer as a blob, one end portion of the blob of the pulp fibers is present in the pulp fiber containing layer, and the other end portion of the blob of the pulp fibers is in contact with the absorbent core. Further, in a case in which a single pulp fiber penetrates the first hydrophilic fiber containing layer so as to be in contact with the absorbent core, one end portion of the single pulp fiber is present in the pulp fiber containing layer, and the other end portion thereof is in contact with the absorbent core.

From the viewpoint of the effect of the present disclosure, to be more specific, from the viewpoint of being difficult to inhibit the diffusing property of the first hydrophilic fiber containing layer in the plane direction, a single pulp fiber preferably penetrates the first hydrophilic fiber containing layer. Further, from the viewpoint of improving the absorbability in the thickness direction of the absorbent body, the pulp fibers as a blob preferably penetrate the first hydrophilic fiber containing layer.

In the absorbent body of the present disclosure, the core wrapping sheet may be disposed at arbitrary surfaces of the absorbent core, and the core wrapping sheet need not cover the entire surface of the absorbent core.

In the absorbent body of the present disclosure, the core wrapping sheet preferably covers at least a portion of the skin side surface of the absorbent core, and more preferably covers the entire surface of the skin side surface of the absorbent core. By the core wrapping sheet being disposed in the skin side surface of the absorbent core, the liquid can be diffused in the plane direction of the absorbent body before the absorbed liquid reaches the absorbent core.

In the absorbent body of the present disclosure, the core wrapping sheet preferably covers at least a portion of the non-skin side surface of the absorbent core, and more preferably covers the entire surface of the non-skin side surface of the absorbent core. This is because, when the absorbent body absorbs a large amount of liquid is supplied at once, since the absorbed liquid reaches the non-skin side surface of the absorbent core, the non-skin side surface of the absorbent core is preferably capable of diffusing the absorbed liquid in the plane direction of the absorbent body.

In the absorbent body of the present disclosure, the core wrapping sheet has a basis weight of, preferably 30 g / m$^2$ to 100 g / m$^2$, more preferably 40 g / m$^2$ to 80 g / m$^2$, and even more preferably 40 g / m$^2$ to 60 g / m$^2$. This is from the viewpoint of securing the diffusing property in the plane direction in the core wrapping sheet, the temporarily storing property of the liquid in the pulp fiber containing layer, and the transferring property of the liquid to the absorbent core.

In the present disclosure, a basis weight is measured as follows.

Ten pieces of samples of a size of 100 mm × 100 mm are taken, the mass of each of the samples is measured, and then the mass (g) of each of the samples is divided by the area (m$^2$) of each of the samples, whereby the basis weight of each of the samples is calculated. The average value of the basis weights of the total ten samples is calculated, and the average value is adopted as the basis weight.

In the absorbent body of the present disclosure, the core wrapping sheet has a density of, preferably 0.05 g / cm$^3$ to 0.20 g / cm$^3$, more preferably 0.06 g / cm$^3$ to 0.15 g / cm$^3$, and even more preferably 0.07 g / cm$^3$ to 0.12 g / cm$^3$. This is from the viewpoint of capillary force of the core wrapping sheet. To be more specific, the core wrapping sheet having the capillary force caused by the above mentioned density range is excellent in drawing liquid to the core wrapping sheet and in delivering the liquid from the core wrapping sheet to the absorbent core, and the absorbent body of the present disclosure, as well as the absorbent article which includes the absorbent body is excellent in rewetting property.

In the present disclosure, a density of a sample is calculated by dividing the basis weight of the sample by the thickness thereof. Incidentally, the measurement method of the basis weight is described above.

In the present disclosure, the measurement method of a thickness of a sample differs depending on whether the sample is an absorbent body or a core wrapping sheet.

The thickness of an absorbent body is measured by PEACOCK Dial Thickness Gauge J-B (diameter of probe : 50 mm, pressure : 294 Pa) manufactured by OZAKI MFG. CO. LTD.

The thickness of a core wrapping sheet is measured by PEACOCK Dial Gauge No.307 (diameter of probe : 44 mm, pressure : 294 Pa) manufactured by OZAKI MFG. CO. LTD.

In the absorbent body of the present disclosure, the absorbent core may be composed by polymer absorbing agents. As the above mentioned polymer absorbing agents, polymer absorbing agents known in the technical field are included, and for example, starch based, cellulose based, or synthetic polymer based polymer absorbing agents may be mentioned. As the starch based or cellulose based polymer absorbing agents, for example, a starch-acrylic acid (salt) graft copolymer, a saponified product of a starch-acrylonitrile copolymer, a crosslinked product of sodium carboxymethyl cellulose, etc., may be mentioned, and as the synthetic polymer based polymer absorbing agents, for example, polyacrylate based, polysulfonate based, maleic anhydride based, polyacrylamide based, polyvinyl alcohol based, polyethylene oxide based, polyaspartate based, polyglutamate based, polyalginate based, starch based, cellulose based, etc., super absorbent polymers (SAP), etc., may be mentioned, and from the viewpoint of the effect of the present disclosure, polyacrylate based (especially, sodium polyacrylate based) super absorbent polymers are preferable.

In the absorbent body of the present disclosure, the absorbent core may include arbitrary components, for example, fibers such as pulp fibers, other than the polymer absorbing agents. The absorbent core may include the above mentioned fibers as a layer, such as a fabric (for example, a nonwoven fabric, a woven fabric, a knitted fabric, etc.). The above mentioned fabric is preferably a hydrophilic fabric from the viewpoint of absorbability of liquid.

In the absorbent body of the present disclosure, in a case in which the absorbent core includes the above mentioned fabric, the absorbent core preferably includes the polymer absorbing agent layer in the skin side surface, the polymer absorbing agent layer in the non-skin side surface, and a fabric layer therebetween. This is because, the blocking of the polymer absorbing agents is suppressed, and the pulp fibers of the pulp fiber containing layer in the core wrapping sheet can be in direct contact with the polymer absorbing agents.

As the pulp fibers which may be included in the absorbent core, those similar to the pulp fibers of the pulp fiber containing layer in the core wrapping sheet may be mentioned.

In the absorbent body of the present disclosure, in an embodiment in which the absorbent core includes the polymer absorbing agents and other arbitrary components, the absorbent core may be a mixture of the polymer absorbing agents and other arbitrary components, for example, pulp fibers, etc. In such cases, the pulp fibers which penetrate the first hydrophilic fiber containing layer transfer the liquid to the polymer absorbing agents directly, or indirectly through the other arbitrary components.

In the absorbent body of the present disclosure, in a case in which the absorbent core includes the polymer absorbing agents and a layer of other arbitrary components, the polymer absorbing agents are preferably present in the surface of the absorbent core, and the pulp fibers which penetrate the first hydrophilic fiber containing layer are preferably in direct contact with the polymer absorbing agents. This is because the liquid which is temporarily stored in the pulp fiber containing layer of the core wrapping sheet can be quickly supplied to the polymer absorbing agents in response to the requirement of the polymer absorbing agents.

In the absorbent body of the present disclosure, in a case in which the polymer absorbing agents are present in the surface of the absorbent core, the first hydrophilic fiber containing layer is preferably fixed to the polymer absorbing agents in the surface of the absorbent core through an adhesion layer. Accordingly, even when the absorbent body is deformed when being worn, the connection between the polymer absorbing agents and the pulp fiber containing layer through the pulp fibers can be retained. As a result, even when the wearer moves intensely, the liquid which has been temporarily stored in the pulp fiber containing layer is directly absorbed by the polymer absorbing agents through the pulp fibers which penetrate the first hydrophilic fiber containing layer in response to the requirement of the polymer absorbing agents, whereby the absorbent body is excellent in absorbing liquid.

In the absorbent body of the present disclosure, in a case in which the core wrapping sheet is disposed at least on the skin surface side of the absorbent core, in the core wrapping sheet which is disposed on the skin surface side of the absorbent core, the portion of the pulp fibers in the pulp fiber containing layer preferably penetrates the second hydrophilic fiber containing layer and reaches the surface of the second hydrophilic fiber containing layer on the opposite side of the pulp fiber containing layer. This is because, in the absorbent article, the pulp fibers which penetrate the second hydrophilic fiber containing layer can quickly draw the liquid which has reached the liquid permeable sheet to the pulp fiber containing layer in the core wrapping sheet so that the liquid can be temporarily stored.

The pulp fibers which penetrate the second hydrophilic fiber containing layer may, as a blob of the pulp fibers, that is, as a plurality of pulp fibers integrally, penetrate the second hydrophilic fiber containing layer and reach the surface of the second hydrophilic fiber containing layer on the opposite side of the pulp fiber containing layer, or as in the first embodiment, a single pulp fiber may penetrate the second hydrophilic fiber containing layer and reach the surface of the second hydrophilic fiber containing layer on the opposite side of the pulp fiber containing layer. From the viewpoint of being difficult to inhibit the diffusing property of the second hydrophilic fiber containing layer in the plane direction, a single pulp fiber preferably penetrates the second hydrophilic fiber containing layer. Further, from the viewpoint of improving the absorbability in the thickness direction of the absorbent body, the pulp fibers as a blob preferably penetrate the second hydrophilic fiber containing layer.

In the absorbent body of the present disclosure, the core wrapping sheet has an absorption height in a water absorption test according to the Klemm method, preferably of 120 mm or higher, more preferably of 130 mm or higher, even more preferably of 140 mm or higher, and still even more preferably of 150 mm or higher. This is from the viewpoint of securing the diffusing property in the plane direction in the core wrapping sheet. Incidentally, in the water absorption test according to the Klemm method, the upper limit in measuring the absorption height is 200 mm, and in the absorbent body of the present disclosure, the upper limit of the absorption height of the core wrapping sheet, in the water absorption test according to the Klemm method, is 200 mm.

In the absorbent body of the present disclosure, the core wrapping sheet has an absorption magnification, in the water absorption test according to the Klemm method, preferably of 2.8 times or more, more preferably of 3.1 times or more, and even more preferably of 3.2 times or more. This is from the viewpoint of securing the temporarily storing property of the liquid in the core wrapping sheet. Further, in the absorbent body of the present disclosure, the core wrapping sheet has an absorption magnification, in the water absorption test according to the Klemm method, preferably of 6.0 times or less. This is from the viewpoint of securing the absorption speed in the absorbent body.

In the present disclosure, the water absorption test according to the Klemm method is measured in accordance with the water absorption test of the Klemm method described in JIS P 8141 : 2004, and the specific procedures thereof are as follows.

(1) A sample is cut into a size of 230 mm × 25 mm (the length × the width), and the initial mass thereof : $W_0$ (g) is measured.

(2) Artificial urine is filled up to a height of 35 mm in a rectangular parallelepiped dipping container with a size of 170 mm × 90 mm × 40mm (the length × the width × the depth). Incidentally, the artificial urine is prepared by dissolving 200 g of urea, 80 g of sodium chloride, 8 g of magnesium sulfate, 3 g of calcium chloride and approximately 1 g of dye : blue No. 1 into 10 L of ion exchanged water.

(3) The sample is fixed to a hanging tool, the lower end in the longitudinal direction : 30 mm is immersed in the artificial urine, and is left for five minutes.

(4) Five minutes later, the height to which the artificial urine ascended is measured as the absorption height.

(5) Subsequently, the sample is removed from the hanging tool, the portion of 30 mm in length which has been immersed in the artificial urine is cut off, and the mass of the sample : $W_1$ (g) is measured.

(6) The absorption magnification : X is calculated in accordance with the following formula.

$$X = [(W_1 \times 230 / 200) - W_0] / W_0$$

(7) The above experiment is repeated for five times, and the average value is adopted.

In the absorbent body of the present disclosure, the core wrapping sheet has, in a diffusion - transfer test, a diffusion length preferably of 130 mm or more, more preferably of 140 mm or more, and even more preferably of 150 mm or more. This is from the viewpoint of diffusing the liquid in the plane direction in the absorbent body.

Incidentally, the diffusion length in the diffusion - transfer test has an upper limit of the measurement of 200 mm, and in the absorbent body of the present disclosure, the upper limit of the diffusion length, in the diffusion - transfer test, is 200 mm.

In the absorbent body of the present disclosure, the core wrapping sheet has, in a diffusion - transfer test, a transfer amount on the first hydrophilic fiber containing layer side preferably of 31.0 g or more, more preferably of 32.0 g or more, even more preferably of 33.0 g or more, and still even more preferably of 34.0 g or more. This is from the viewpoint of transferring the liquid in the thickness direction in the absorbent body of the present disclosure.

Incidentally, the transfer amount is an indication to indirectly grasp the amount of the pulp fibers in the pulp fiber containing layer which penetrate the first hydrophilic fiber containing layer of the core wrapping sheet.

In the absorbent body of the present disclosure, in a case in which the portion of the pulp fibers in the pulp fiber containing layer preferably penetrates the second hydrophilic fiber containing layer and reaches the surface of the second hydrophilic fiber containing layer on the opposite side of the pulp fiber containing layer, the core wrapping sheet has, in the diffusion - transfer test, the transfer amount on the second hydrophilic fiber containing layer side preferably of 31.0 g or more, more preferably of 32.0 g or more, even more preferably of 33.0 g or more, and still even more preferably of 34.0 g or more. This is from the viewpoint of, while quickly drawing the liquid which is present in the layer adjacent to the second hydrophilic fiber containing layer (for example, the liquid permeable sheet of the absorbent article) into the second hydrophilic fiber containing layer, diffusing the liquid in the plane direction of the second hydrophilic fiber containing layer, and further, quickly delivering the liquid to the pulp fiber containing layer.

Incidentally, the transfer amount is an indication to indirectly grasp the amount of the pulp fibers in the pulp fiber containing layer which penetrate the second hydrophilic fiber containing layer of the core wrapping sheet.

The above mentioned diffusion - transfer test is performed as follows.

(1) 20 pieces of filter paper with the respective sizes of 200 mm $\times$ 50 mm are stacked so that the edges thereof are aligned, whereby a filter paper set is formed. The mass : $F_0$ (g) of the filter paper set before the test is measured. Incidentally, four pieces of filter paper with the respective sizes of 50 mm $\times$ 50 mm may be disposed so as to be adjacent to each other, whereby may be handled as a filter paper with a size of 200 mm $\times$ 50 mm.

(2) A sample is cut into a size of 250 mm $\times$ 50 mm.

(3) The filter paper set is disposed on an acrylic board, and the above mentioned sample is stacked on the filter paper set so that the edges of the 200 mm $\times$ 50 mm sized measurement region of the sample are aligned to the edges of the filter paper set. The sample is evaluated in two patterns, of the case in which the sample is disposed so that the first hydrophilic fiber containing layer is adjacent to the filter paper set, and of the case in which the sample is disposed so that the second hydrophilic fiber containing layer is adjacent to the filter paper set. Incidentally, the remaining 50 mm $\times$ 50 mm sized portion of the sample is immersed in the artificial urine as an immersion region.

(4) A transparent acrylic plate with a size of 250 mm $\times$ 80 mm (mass : 12.8 g) is disposed on the measurement region of the sample, and a transparent glass plate with a size of 300 mm $\times$ 50 mm (mass : 174.0 g) is disposed on the transparent acrylic board.

(5) The portion of 30 mm $\times$ 50 mm among the immersion region of the sample which is distant from the filter paper is immersed in the artificial urine. Incidentally, the composition of the artificial urine is described above.

(6) After 10 minutes of immersion, the diffusion length of the artificial urine in the measurement region of the sample is calculated through the transparent glass plate and the transparent acrylic plate. Incidentally, the as the diffusion length, the average value of the one measured in which the sample is disposed so that the first hydrophilic fiber containing layer is in contact with the filter paper set (the diffusion length on the first hydrophilic fiber containing layer side) and the one measured in which the sample is disposed so that the second hydrophilic fiber containing layer is in contact with the filter paper set (the diffusion length on the second hydrophilic fiber containing layer side) is adopted.

(7) Subsequently, the transparent glass plate, the transparent acrylic plate, and the sample are removed, and the mass : $F_1$ (g) of the filter paper set after the test is measured.

(8) The transfer amount is calculated by the following formula.

$$\text{Transfer amount (g)} = F_1(g) - F_0(g)$$

Incidentally, in the transfer amount, the one measured in which the sample is disposed so that the first hydrophilic fiber containing layer is in contact with the filter paper set (the transfer amount on the first hydrophilic fiber containing layer side) and the one measured in which the sample is disposed so that the second hydrophilic fiber containing layer is in contact with the filter paper set (the transfer amount on the second hydrophilic fiber containing layer side) are distinguished from each other.

(9) The measurement is repeated for five times, and the average values of the respective diffusion lengths, the transfer amount on the first hydrophilic fiber containing layer side, and the transfer amount on the second hydrophilic fiber containing layer side are calculated.

Incidentally, the above mentioned diffusion - transfer test is performed in a constant temperature and humidity chamber of 20 ± 5 °C × 65 ± 5 %RH

In the absorbent body of the present disclosure, the manufacturing method of the core wrapping sheet is not particularly limited, however, the core wrapping sheet may be manufactured as follows.

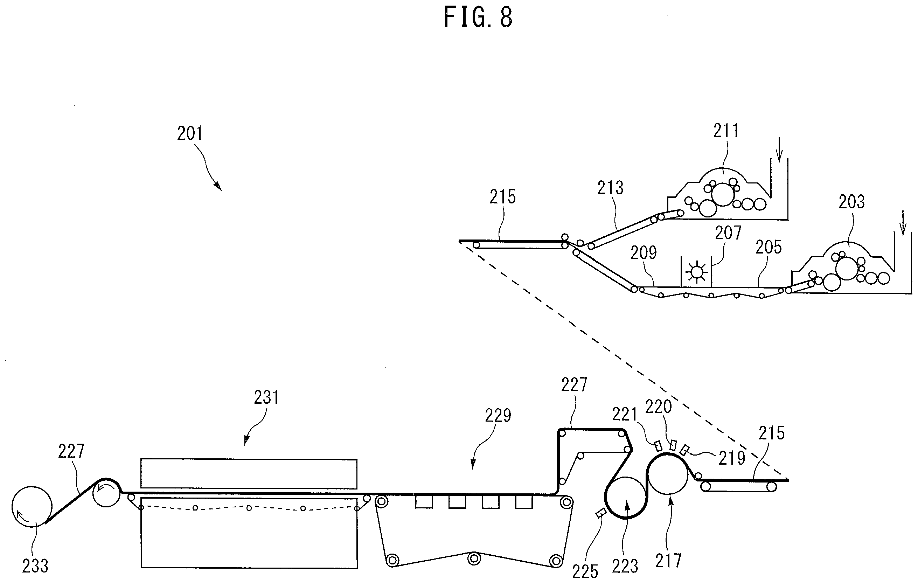

FIG. 8 is a schematic view of a manufacturing apparatus 201 which may be used in the manufacturing method of the above mentioned core wrapping sheet.

In the manufacturing apparatus 201 as shown in FIG. 8, fibers which configure the first hydrophilic fiber containing layer which has been prepared by a method known in the technical field, for example, the first hydrophilic fibers, are fed from a feeder to a carding device 203, so as to form a web 205. Subsequently, fibers which configure the pulp fiber containing layer which has been prepared by a method known in the technical field, for example, the pulp fibers, are deposited from a feeder 207 on the web 205, so as to form a laminated layer web 209.

In parallel with the formation of the laminated layer web 209, fibers which configure the second hydrophilic fiber containing layer which has been prepared by a method known in the technical field, for example, the second hydrophilic fibers, are fed from a feeder to a carding device 211, so as to form a web 213. Subsequently, the web 213 is laminated on the laminated layer web 209, so as to form a laminated layer web 215.

Subsequently, the laminated layer web 215 is subjected to a water jet processing, so as to form a core wrapping sheet 227. The water jet processing is performed by jetting fluid onto the laminated layer web 215 on the second hydrophilic fiber containing layer side (the first water jet processing), followed by jetting fluid onto the laminated layer web 215 on the first hydrophilic fiber containing layer side (the second water jet processing).

The first water jet processing is performed by a first suction drum 217 which sucks the laminated layer web 215 which is being conveyed while rotating around the shaft axis, from the first hydrophilic fiber containing layer side, and retains the laminated layer web 215 on the outer circumferential surface thereof, and a first fluid jetting device 219, a second fluid jetting device 220, and a third fluid jetting device 221 which jet fluid to the laminated layer web 215 retained on the outer circumferential surface of the first suction drum 217, from the second hydrophilic fiber containing layer side.

The second water jet processing is performed by a second suction drum 223 which sucks the laminated layer web 215 which is being conveyed while rotating around the shaft axis, from the second hydrophilic fiber containing layer side, and retains the laminated layer web 215 on the outer circumferential surface thereof, and a fourth fluid jetting device 225 which jets fluid to the laminated layer web 215 retained on the outer circumferential surface of the second suction drum 223, from the first hydrophilic fiber containing layer side.

In the first water jet processing, the fluid jetting pressures of the first fluid jetting device 219, the second fluid jetting device 220, and the third fluid jetting device 221, and the suction pressure of the first suction drum 217 are adjusted (for example, the fluid jetting pressure of the third fluid jetting device 221 is adjusted to a pressure which is higher than that normally performed in the technical field), whereby the degree of penetration of the pulp fibers in the pulp fiber containing layer with respect to the first hydrophilic fiber containing layer can mainly be adjusted. In the same manner, in the second water jet processing, the fluid jetting pressure of the fourth fluid jetting device 225 and the suction pressure of the second suction drum 223 are adjusted (for example, the fluid jetting pressure of the fourth fluid jetting device 225 is adjusted to a pressure which is higher than that normally performed in the technical field), whereby the degree of penetration of the pulp fibers in the pulp fiber containing layer with respect to the second hydrophilic fiber containing layer can mainly be adjusted.

Subsequently, the core wrapping sheet 227 is moved to a dehydration device 229 which is known in the technical field, and the moisture of the core wrapping sheet 227 is dehydrated. Subsequently, the core wrapping sheet 227 is moved to a drying device 231 which is known in the technical field, and is wound up by a roll 233. The core wrapping sheet 227 which is wound up by the roll 233 is cut by a method which is known in the technical field at the time of manufacturing the absorbent article, whereby the absorbent body is manufactured.

The absorbent body of the present disclosure is an absorbent body for an absorbent article. As an absorbent article which includes the absorbent body of the present disclosure, an absorbent article which includes a liquid permeable sheet, a liquid permeable sheet, and the absorbent body between the liquid permeable sheet and the liquid impermeable sheet, may be mentioned.

The above mentioned absorbent article is not particularly limited, and for example, an absorbent article which mainly absorbs urine, such as a disposable diaper, a urine absorbing pad, a urinating sheet for animals, etc., an absorbent article which mainly absorbs menstrual blood, etc., such as a sanitary napkin, a panty liner, etc., may be mentioned. The absorbent article for which the absorbent body of the present disclosure may be used is preferably an absorbent article which mainly absorbs urine, from the viewpoint that the effect of the present disclosure can be more exhibited.

Examples

Hereinbelow, the present disclosure is explained with examples, however, the present disclosure is not limited to these examples.

[Manufacturing example 1]

- Manufacturing of the core wrapping sheet No. 1 -

By using the manufacturing apparatus described in FIG. 8, the core wrapping sheet No. 1 was manufactured. In the core wrapping sheet No. 1, the first hydrophilic fiber containing layer was configured by 100 mass % of rayon fibers (a fiber diameter : 1.7 dtex, an average fiber length : 40 mm), the second hydrophilic fiber containing layer was configured by 100 mass % of rayon fibers (a fiber diameter : 1.7 dtex, an average fiber length : 40 mm), and the pulp fiber containing layer was configured by 100 mass % of coniferous pulp fibers (an average fiber length : 3mm). Further, in the core wrapping sheet No. 1, the first hydrophilic fiber containing layer, the pulp fiber containing layer, and the second hydrophilic fiber containing layer were supplied so that the mass ratio thereof was 1 : 3 : 1. The basis weight, the thickness, and the density of the core wrapping sheet No. 1 are shown in Table 1.

[Manufacturing example 2]

- Manufacturing of the core wrapping sheet No. 2 -

In the same manner as the Manufacturing example 1, the core wrapping sheet No. 2 was manufactured in which the basis weight, the thickness, and the density were different from those of the core wrapping sheet No. 1. The basis weight, the thickness, and the density of the core wrapping sheet No. 2 are shown in Table 1.

[Manufacturing example 3]

- Manufacturing of the core wrapping sheet No. 3 -

The core wrapping sheet No. 3 was manufactured according to the Manufacturing example 1, except for changing the fibers which configure each of the first hydrophilic fiber containing layer and the second hydrophilic fiber containing layer to a mixture of 70 mass % of rayon fibers (a fiber diameter : 1.7 dtex, an average fiber length : 40 mm) and 30 mass % of polyethylene terephthalate (PET) fibers (a fiber diameter : 1.6 dtex, an average fiber length : 44 mm). In the core wrapping sheet No. 3, the first hydrophilic fiber containing layer, the pulp fiber containing layer, and the second hydrophilic fiber containing layer were supplied so that the mass ratio thereof is 1 : 3 : 1. The basis weight, the thickness, and the density of the core wrapping sheet No. 3 are shown in Table 1.

[Manufacturing example 4]

- Manufacturing of the core wrapping sheet No. 4 -

A spun lace nonwoven fabric which was configured by 100 mass % of rayon fibers (a fiber diameter : 1.4 dtex, an average fiber length : 44 mm) was manufactured. The basis weight, the thickness, and the density of the core wrapping sheet No. 4 are shown in Table 1.

[Manufacturing example 5]

- Manufacturing of the disposable diaper No. 1 -

An absorbent core No. 1 was formed on the core wrapping sheet No. 1, and the core wrapping sheet No. 1 was stacked thereon, whereby the absorbent body No. 1 was formed.
To be more specific, a hot melt adhesive agent was applied on the core wrapping sheet No. 1, and polyacrylate based super absorbent polymers (SAP) were stacked so that the basis weight thereof was 125 g / m$^2$, whereby a SAP layer was formed. On the SAP layer, a hydrophilic nonwoven fabric, on both surfaces of which a hot melt adhesive agent was applied (polyethylene fibers : 40 mass %, polyethylene terephthalate fibers : 60 mass %, a basis weight : 40 g / m$^2$, a hydrophilic agent is applied) was stacked. On the hydrophilic nonwoven fabric, polyacrylate based super absorbent polymers (SAP) were stacked so that the basis weight thereof was 125 g / m$^2$, whereby a SAP layer was formed. On the SAP layer, the core wrapping sheet No. 1 to which a hot melt adhesive agent was applied was stacked, and the thickness was adjusted, whereby the absorbent body No. 1 was formed. The absorbent body No. 1 had a basis weight of 430 g / m$^2$, and a thickness of 2.14 mm. When the cross section of the absorbent body No. 1 was viewed by an electron microscope, it was confirmed that the pulp fibers in the pulp fiber containing layer penetrated the first hydrophilic fiber containing layer and were in direct contact with the polyacrylate based super absorbent polymers in the absorbent core No. 1.
By using the absorbent body No. 1, the disposable diaper No. 1 as shown in FIG. 1 was manufactured.

[Manufacturing example 6]

- Manufacturing of the disposable diaper No. 2 -

An absorbent body No. 2 and the disposable diaper No. 2 were manufactured according to the Manufacturing example 5, except for changing the core wrapping sheet No. 1 so as to be replaced to the core wrapping sheet No. 2. The absorbent body No. 2 had a basis weight of 430 g / m$^2$, and a thickness of 2.46 mm.
Incidentally, when the cross section of the absorbent body No. 2 was viewed by an electron microscope, it was confirmed that the pulp fibers in the pulp fiber containing layer penetrated the first hydrophilic fiber containing layer and were in direct contact with the polyacrylate based super absorbent polymers in the absorbent core No. 1.

[Manufacturing example 7]

- Manufacturing of the disposable diaper No. 3 -

An absorbent body No. 3 and the disposable diaper No. 3 were manufactured according to the Manufacturing example 5, except for changing the core wrapping sheet No. 1 so as to be replaced to the core wrapping sheet No. 3. The absorbent body No. 3 had a basis weight of 420 g / m$^2$, and a thickness of 1.77 mm.
Incidentally, when the cross section of the absorbent body No. 3 was viewed by an electron microscope, it was confirmed that the pulp fibers in the pulp fiber containing layer penetrated the first hydrophilic fiber containing layer and were in direct contact with the polyacrylate based super absorbent polymers in the absorbent core No. 1.

[Manufacturing example 8]

- Manufacturing of the disposable diaper No. 4 -

An absorbent core No. 2 was formed on the core wrapping sheet No. 1, and the core wrapping sheet No. 1 was stacked thereon, whereby the absorbent body No. 4 was formed.
To be more specific, a hot melt adhesive agent was applied on the core wrapping sheet No. 1, and polyacrylate based super absorbent polymers (SAP) were stacked thereon so that the basis weight thereof was 250 g / m$^2$, the core

wrapping sheet No. 2 to which a hot melt adhesive agent was applied was stacked on the SAP layer, and the thickness of the stack was adjusted, whereby the absorbent body No. 4 was formed. The absorbent body No. 4 had a basis weight of 375 g / m$^2$, and a thickness of 1.26 mm.

Incidentally, when the cross section of the absorbent body No. 4 was viewed by an electron microscope, it was confirmed that the pulp fibers in the pulp fiber containing layer penetrated the first hydrophilic fiber containing layer and were in direct contact with the polyacrylate based super absorbent polymers.

The disposable diaper No. 4 was manufactured in the same manner as the Manufacturing example 5 except for changing the absorbent body No. 1 so as to be replaced to the absorbent body No. 4.

[Manufacturing example 9]

- Manufacturing of the disposable diaper No. 5 -

An absorbent body No. 5 and the disposable diaper No. 5 were manufactured according to the Manufacturing example 5, except for changing the core wrapping sheet No. 1 so as to be replaced to the core wrapping sheet No. 4. The absorbent body No. 5 had a basis weight of 420 g / m$^2$, and a thickness of 1.83 mm.

[Examples 1 to 4 and Comparative Example 1

An absorption test defined as follows was performed for the disposable diapers No. 1 to No. 5, and an absorption time, rewetting amount, and a diffusion length on the liquid impermeable sheet were evaluated. The results are shown in Table 1.

[Absorption test]

(1) An absorbent article is set on a U-shaped instrument a side view of which is substantially U-shaped. Incidentally, the absorbent article is set on the U-shaped instrument so that the central position in the longitudinal direction of the absorbent body and the central portion of the U-shaped instrument (the position at which the height is the lowest) are matched.

<First cycle>

(2) 80mL of artificial urine (the first time) is injected from a burette at a speed of 80 mL / 10 sec to the central position of the absorbent body.

(3) The time from the start of the artificial urine injection of the first time until the artificial urine in the U-shaped instrument disappears is recorded as the absorption time (80 mL).

(4) After three minutes from the start of the artificial urine injection of the first time, the outline (80 mL) of the region in which the artificial urine has diffused in the liquid impermeable sheet of the absorbent article is recorded.

(5) After five minutes from the start of the artificial urine injection of the first time, filter paper of approximately 60 g with a size of 100 mm × 100 mm is placed on the liquid permeable sheet of the absorbent article with the artificial urine injection point as the center. Further, a weight of 3.5 kg with a size of 100 mm × 100 mm × 50 mm (height) is placed thereon. Incidentally, the mass of the filter paper is measured before being placed on the liquid permeable sheet.

(6) After eight minutes from the start of the artificial urine injection of the first time, the weight is removed, the mass of the filter paper is measured, the mass of the filter paper before the test is subtracted therefrom, and the difference is regarded as the rewetting amount (80 mL) .

<Second cycle>

(7) After ten minutes from the start of the artificial urine injection of the first time, 80mL of artificial urine (the second time) is injected from a burette at a speed of 80 mL / 10 sec to the central position of the absorbent body.

(8) The time from the start of the artificial urine injection of the second time until the artificial urine in the U-shaped instrument disappears is recorded as the absorption time (160 mL).

(9) After three minutes from the start of the artificial urine injection of the second time, the outline (160 mL) of the region in which the artificial urine has diffused in the liquid impermeable sheet of the absorbent article is recorded.

(10) After five minutes from the start of the artificial urine injection of the second time, filter paper of approximately 60 g with a size of 100 mm × 100 mm is placed on the liquid permeable sheet of the absorbent article with the artificial urine injection point as the center. Further, a weight of 3.5 kg with a size of 100 mm × 100 mm × 50 mm (height) is placed thereon. Incidentally, the mass of the filter paper is measured before being placed on the liquid permeable sheet.

(11) After eight minutes from the start of the artificial urine injection of the second time, the weight is removed, the mass of the filter paper is measured, the mass of the filter paper before the test is subtracted therefrom, and the difference is regarded as the rewetting amount (160 mL).

<Third cycle>

(12) The operations of (7) to (11) are repeated, the absorption time (240 mL) is measured, and the outline (240 mL) is recorded.

(13) From the outlines (80, 160, 240 mL) of the region in which the artificial urine has diffused, the diffusion lengths (80, 160, 240 mL) of the artificial urine in the longitudinal direction of the absorbent article is measured.

Incidentally, the composition of the artificial urine is described above.

[Table 1]

| Example No. | | Example 1 | Example 2 | Example 3 | Example 4 | Comparative Example 1 |
|---|---|---|---|---|---|---|
| Disposable diaper No. | | No. 1 | No. 2 | No. 3 | No. 4 | No. 5 |
| Absorbent body No. | | No. 1 | No. 2 | No. 3 | No. 4 | No. 5 |
| • Basis weight | g / m$^2$ | 430 | 430 | 420 | 375 | 420 |
| • Thickness | mm | 2.14 | 2.46 | 1.77 | 1.26 | 1.83 |
| Core wrapping sheet No. | | No. 1 | No. 2 | No. 3 | No. 1 | No. 4 |
| • First hydrophilic fiber containing layer | | Rayon | Rayon | Rayon + PET | Rayon | Rayon |
| • Pulp fiber containing layer | | Pulp fibers | Pulp fibers | Pulp fibers | Pulp fibers | |
| • Second hydrophilic fiber containing layer | | Rayon | Rayon | Rayon + PET | Rayon | |
| • Basis weight | g / m$^2$ | 56 | 56 | 50 | 56 | 51 |
| • Thickness | mm | 0.30 | 0.65 | 0.38 | 0.30 | 0.53 |
| • Density | g / cm$^3$ | 0.19 | 0.09 | 0.13 | 0.19 | 0.10 |
| Absorbent core No. | | No. 1 | No. 1 | No. 1 | No. 2 | No. 1 |
| • Configuration (Upper layer/ Middle layer/ Lower layer | | SAP/ hydrophilic nonwoven fabric/ SAP | SAP/ hydrophilic nonwoven fabric/ SAP | SAP/ hydrophilic nonwoven fabric/ SAP | SAP | SAP/ hydrophilic nonwoven fabric/ SAP |
| • Basis weight | g / m$^2$ | 125/ 40/ 125 | 125/ 40/ 125 | 125/ 40/ 125 | 250 | 125/ 40/ 125 |

(continued)

| Example No. | | Example 1 | Example 2 | Example 3 | Example 4 | Comparative Example 1 |
|---|---|---|---|---|---|---|
| Absorption time / sec | 80 mL | 34 | 30 | 31 | 33 | 32 |
| | 160 mL | 20 | 27 | 21 | 27 | 37 |
| | 240 mL | 41 | 42 | 37 | 50 | 70 |
| Rewetting amount / g | 80 mL | 0.2 | 0.2 | 0.2 | 0.3 | 0.2 |
| | 160 mL | 6.9 | 1.2 | 11.5 | 6.1 | 6.0 |
| | 240 mL | 38.0 | 26.0 | 41.0 | 34.0 | 40.0 |
| Diffusion length / mm | 80 mL | 253 | 237 | 237 | 230 | 243 |
| | 160 mL | 273 | 248 | 248 | 248 | 243 |
| | 240 mL | 326 | 296 | 296 | 299 | 273 |

From Table 1, it can be understood that in the disposable diapers No. 1 to No. 4 which respectively include the absorbent bodies No. 1 to No. 4 of the present disclosure, the absorption time is shorter and the diffusion length is longer when the artificial urine is absorbed for a plurality of times, compared to the disposable diaper No. 5 which includes the conventional diffusion sheet. Further, when the disposable diaper No. 2 and the disposable diaper No. 5 are compared in which the densities of the core wrapping sheets are equivalent, it can be understood that in the disposable diaper No. 2, the rewetting amount is less when the artificial urine is absorbed for a plurality of times than that of the disposable diaper No. 5.

[Example 5 and Comparative Example 2]

The diffusion length and the transfer amount on the first hydrophilic fiber containing layer, according to the diffusion - transfer test, are measured for the core wrapping sheet No. 2 and the core wrapping sheet No. 4. The results are shown in Table 2.

[Table 2]

| Core wrapping sheet No. | | No. 2 | No. 4 |
|---|---|---|---|
| • First hydrophilic fiber containing layer | | Rayon | Rayon |
| • Pulp fiber containing layer | | Pulp fibers | |
| • Second hydrophilic fiber containing layer | | Rayon | |
| • Basis weight | g / m$^2$ | 56 | 51 |
| • Thickness | mm | 0.65 | 0.53 |
| • Density | g / cm$^3$ | 0.09 | 0.10 |
| Diffusion length on first hydrophilic fiber containing layer side / mm | | 152 | 123 |
| Transfer amount / g | | 35.8 | 29.7 |

Reference Signs List

1 absorbent article
3 liquid permeable sheet
5 liquid impermeable sheet
7 absorbent body
11 absorbent core
13, 14 core wrapping sheet

15 skin side surface
17 non-skin side surface
19 polymer absorbing agent
20, 20a, 20b polymer absorbing agent layer
21 hydrophilic fabric
22 layer
23, 24 first hydrophilic fiber
25, 26 first hydrophilic fiber containing layer
27, 28 pulp fiber
29, 30 pulp fiber containing layer
31, 32 second hydrophilic fiber
33, 34 second hydrophilic fiber containing layer
35, 36 surface
41 liquid
43 supply point
45 liquid permeation region
101 leakage prevention barrier
103 elastic member
105 fixed portion
107 elastic member
109 tape fastener

**Claims**

1. An absorbent body for an absorbent article, comprising an absorbent core which includes a polymer absorbing agent and further comprising a core wrapping sheet, wherein

   the core wrapping sheet includes (i) a first hydrophilic fiber containing layer which is adjacent to the absorbent core and includes first hydrophilic fibers with an average fiber length of 25 mm to 75 mm, (ii) a pulp fiber containing layer which is adjacent to the first hydrophilic fiber containing layer and includes pulp fibers, and (iii) a second hydrophilic fiber containing layer which is adjacent to the pulp fiber containing layer and includes second hydrophilic fibers with an average fiber length of 25 mm to 75 mm, and
   a portion of the pulp fibers in the pulp fiber containing layer penetrates the first hydrophilic fiber containing layer and is in contact with the absorbent core.

2. The absorbent body according to claim 1, wherein

   the absorbent core includes the polymer absorbing agent in a surface of the absorbent core, and
   the portion of the pulp fibers in the pulp fiber containing layer penetrates the first hydrophilic fiber containing layer and is in contact with the polymer absorbing agent in the surface of the absorbent core.

3. The absorbent body according to claim 2, wherein the first hydrophilic fiber containing layer is fixed to the polymer absorbing agent in the surface of the absorbent core through an adhesion layer.

4. The absorbent body according to any one of claims 1 to 3, wherein

   the absorbent body includes the core wrapping sheet at least on a skin side surface of the absorbent core, and
   in the core wrapping sheet which is disposed in the skin side surface of the absorbent core, the portion of the pulp fibers in the pulp fiber containing layer penetrates the second hydrophilic fiber containing layer and reaches a surface of the second hydrophilic fiber containing layer on an opposite side of the pulp fiber containing layer.

5. The absorbent body according to any one of claims 1 to 4, wherein each of the first hydrophilic fibers and the second hydrophilic fibers are cellulose based fibers.

6. The absorbent body according to any one of claims 1 to 5, wherein the pulp fibers in the pulp fiber containing layer have an average fiber length of 1 mm to 10 mm.

7. The absorbent body according to any one of claims 1 to 6, wherein the core wrapping sheet has a basis weight of 30 g / m$^2$ to 100 g / m$^2$.

8. The absorbent body according to any one of claims 1 to 7, wherein the core wrapping sheet has a density of 0.05 g / cm$^3$ to 0.20 g / cm$^3$.

9. The absorbent body according to any one of claims 1 to 8, wherein the core wrapping sheet has a diffusion length of 130 mm or more, and a transfer amount on a first hydrophilic fiber containing layer side of 31.0 g or more, in a diffusion - transfer test.

10. The absorbent body according to any one of claims 1 to 9, wherein the core wrapping sheet is adjacently disposed in both surfaces of a skin side surface and a non-skin side surface of the absorbent core.

11. The absorbent body according to claim 10, wherein

the absorbent core includes the polymer absorbing agents in the both surfaces of the skin side surface and the non-skin side surface of the absorbent core, and
the pulp fibers in the pulp fiber containing layer of the core wrapping sheet disposed in the skin side surface and the non-skin side surface are in contact with the polymer absorbing agents in the skin side surface and the non-skin side surface, respectively, of the absorbent core.

12. The absorbent body according to claim 11, wherein the absorbent core includes a hydrophilic fabric between the polymer absorbing agent which is present in the skin side surface and the polymer absorbing agent which is present in the non-skin side surface.


**Patentansprüche**

1. Ein absorbierender Körper für einen absorbierenden Artikel, der einen absorbierenden Kern umfasst, der ein polymerabsorbierendes Mittel beinhaltet, und ferner eine Kernverpackungslage umfasst, wobei
die Kernverpackungslage (i) eine erste hydrophile faserhaltige Schicht, die an den absorbierenden Kern angrenzt und erste hydrophile Fasern mit einer durchschnittlichen Faserlänge von 25 mm bis 75 mm umfasst, (ii) eine pulpfaserhaltige Schicht, die an die erste hydrophile faserhaltige Schicht angrenzt und Pulpfasern umfasst, und (iii) eine zweite hydrophile faserhaltige Schicht, die an die pulpfaserhaltige Schicht angrenzt und zweite hydrophile Fasern mit einer durchschnittlichen Faserlänge von 25 mm bis 75 mm umfasst, umfasst und
ein Teil der Pulpfasern in der pulpfaserhaltigen Schicht in die erste hydrophile faserhaltige Schicht eindringt und mit dem absorbierenden Kern in Kontakt steht.

2. Der absorbierende Körper gemäß Anspruch 1, wobei
der absorbierende Kern das polymerabsorbierende Mittel in einer Oberfläche des absorbierenden Kerns umfasst, und
der Teil der Pulpfasern in der pulpfaserhaltigen Schicht in die erste hydrophile faserhaltige Schicht eindringt und mit dem polymerabsorbierenden Mittel in der Oberfläche des absorbierenden Kerns in Kontakt steht.

3. Der absorbierende Körper gemäß Anspruch 2, wobei die erste hydrophile faserhaltige Schicht mittels einer Haftschicht an dem polymerabsorbierenden Mittel in der Oberfläche des absorbierenden Kerns befestigt ist.

4. Der absorbierende Körper gemäß einem der Ansprüche 1 bis 3, wobei
der absorbierende Körper die Kernverpackungslage mindestens auf einer hautseitigen Oberfläche des absorbierenden Kerns umfasst, und
in der Kernverpackungslage, die in der hautseitigen Oberfläche des absorbierenden Kerns angeordnet ist, wobei der Teil der Pulpfasern in der pulpfaserhaltigen Schicht in die zweite hydrophile faserhaltige Schicht eindringt und eine Oberfläche der zweiten hydrophilen faserhaltigen Schicht auf einer gegenüberliegenden Seite der pulpfaserhaltigen Schicht erreicht.

5. Der absorbierende Körper gemäß einem der Ansprüche 1 bis 4, wobei jede der ersten hydrophilen Fasern und der zweiten hydrophilen Fasern Cellulose-basierende Fasern sind.

**6.** Der absorbierende Körper gemäß einem der Ansprüche 1 bis 5, wobei die Pulpfasern in der pulpfaserhaltigen Schicht eine durchschnittliche Faserlänge von 1 mm bis 10 mm aufweisen.

**7.** Der absorbierende Körper gemäß einem der Ansprüche 1 bis 6, wobei die Kernverpackungslage ein Flächengewicht von 30 g/m$^2$ bis 100 g/m$^2$ aufweist.

**8.** Der absorbierende Körper gemäß einem der Ansprüche 1 bis 7, wobei die Kernverpackungslage eine Dichte von 0,05 g/cm$^3$ bis 0,20 g/cm$^3$ aufweist.

**9.** Der absorbierende Körper gemäß einem der Ansprüche 1 bis 8, wobei die Kernverpackungslage eine Diffusionslänge von 130 mm oder mehr und eine Übertragungsmenge auf einer ersten hydrophilen faserhaltigen Schicht von 31,0 g oder mehr in einem Diffusions-Transfer-Test aufweist.

**10.** Der absorbierende Körper gemäß einem der Ansprüche 1 bis 9, wobei die Kernverpackungslage angrenzend in beiden Oberflächen einer hautseitigen Oberfläche und einer nicht hautseitigen Oberfläche des absorbierenden Kerns angeordnet ist.

**11.** Der absorbierende Körper gemäß Anspruch 10, wobei
der absorbierende Kern die polymerabsorbierenden Mittel in den beiden Oberflächen der hautseitigen Oberfläche und der nicht hautseitigen Oberfläche des absorbierenden Kerns umfasst, und
die Pulpfasern in der pulpfaserhaltigen Schicht der in der hautseitigen Oberfläche angeordneten Kernverpackungslage und der nicht hautseitigen Oberfläche mit den polymerabsorbierenden Mitteln in der hautseitigen Oberfläche und der nicht hautseitigen Oberfläche, entsprechend, des Absorptionskerns in Kontakt stehen.

**12.** Der absorbierende Körper gemäß Anspruch 11, wobei der absorbierende Kern ein hydrophiles Gewebe zwischen dem polymerabsorbierenden Mittel, das in der hautseitigen Oberfläche vorhanden ist, und dem polymerabsorbierenden Mittel, das in der nicht hautseitigen Oberfläche vorhanden ist, umfasst.


**Revendications**

**1.** Corps absorbant pour un article absorbant, comprenant un noyau absorbant qui comporte un agent absorbant polymère et comprenant en outre une feuille d'emballage de noyau, dans lequel

la feuille d'emballage de noyau comporte (i) une première couche contenant des fibres hydrophiles qui est adjacente au noyau absorbant et comporte des premières fibres hydrophiles d'une longueur de fibre moyenne de 25 mm à 75 mm, (ii) une couche contenant des fibres de pâte à papier qui est adjacente à la première couche contenant des fibres hydrophiles et comporte des fibres de pâte à papier, et (iii) une seconde couche contenant des fibres hydrophiles qui est adjacente à la couche contenant des fibres de pâte à papier et comporte des secondes fibres hydrophiles avec une longueur de fibre moyenne de 25 mm à 75 mm, et
une partie des fibres de pâte à papier dans la couche contenant des fibres de pâte à papier pénètre dans la première couche contenant des fibres hydrophiles et est en contact avec le noyau absorbant.

**2.** Corps absorbant selon la revendication 1, dans lequel
le noyau absorbant comporte l'agent absorbant polymère dans une surface du noyau absorbant, et
la partie des fibres de pâte à papier dans la couche contenant des fibres de pâte à papier pénètre dans la première couche contenant des fibres hydrophiles et est en contact avec l'agent absorbant polymère dans la surface du noyau absorbant.

**3.** Corps absorbant selon la revendication 2, dans lequel la première couche contenant des fibres hydrophiles est fixée à l'agent absorbant polymère dans la surface du noyau absorbant par l'intermédiaire d'une couche d'adhérence.

**4.** Corps absorbant selon l'une quelconque des revendications 1 à 3, dans lequel le corps absorbant comporte la feuille d'emballage de noyau au moins sur une surface côté peau du noyau absorbant, et
dans la feuille d'emballage de noyau qui est disposée dans la surface côté peau du noyau absorbant, la partie des fibres de pâte à papier dans la couche contenant des fibres de pâte à papier pénètre dans la seconde couche contenant des fibres hydrophiles et atteint une surface de la seconde couche contenant des fibres hydrophiles sur un côté opposé de la couche contenant des fibres de pâte à papier.

**5.** Corps absorbant selon l'une quelconque des revendications 1 à 4, dans lequel chacune des premières fibres hydrophiles et des secondes fibres hydrophiles sont des fibres à base de cellulose.

**6.** Corps absorbant selon l'une quelconque des revendications 1 à 5, dans lequel les fibres de pâte à papier dans la couche contenant des fibres de pâte à papier ont une longueur de fibre moyenne de 1 mm à 10 mm.

**7.** Corps absorbant selon l'une quelconque des revendications 1 à 6, dans lequel la feuille d'emballage de noyau a un poids de base de 30 g/m$^2$ à 100 g/m$^2$.

**8.** Corps absorbant selon l'une quelconque des revendications 1 à 7, dans lequel la feuille d'emballage de noyau a une densité de 0,05 g/cm$^3$ à 0,20 g/cm$^3$.

**9.** Corps absorbant selon l'une quelconque des revendications 1 à 8, dans lequel la feuille d'emballage de noyau a une longueur de diffusion de 130 mm ou plus, et une quantité de transfert sur une première face de couche contenant des fibres hydrophiles de 31,0 g ou plus, dans un test de diffusion - transfert.

**10.** Corps absorbant selon l'une quelconque des revendications 1 à 9, dans lequel la feuille d'emballage de noyau est disposée de manière adjacente dans les deux surfaces d'une surface côté peau et d'une surface non côté peau du noyau absorbant.

**11.** Corps absorbant selon la revendication 10, dans lequel

le noyau absorbant comporte les agents absorbants polymères dans les deux surfaces de la surface côté peau et de la surface non côté peau du noyau absorbant, et

les fibres de pâte à papier dans la couche contenant des fibres de pâte à papier de la feuille d'emballage de noyau disposée dans la surface côté peau et la surface non côté peau sont en contact avec les agents absorbants polymères dans la surface côté peau et la surface non côté peau, respectivement, du noyau absorbant.

**12.** Corps absorbant selon la revendication 11, dans lequel le noyau absorbant comporte un tissu hydrophile entre l'agent absorbant polymère qui est présent dans la surface côté peau et l'agent absorbant polymère qui est présent dans la surface non côté peau.

# FIG. 1

# FIG. 2

EP 3 357 466 B1

# FIG. 3

(a)

(b)

# FIG. 4

(a)

(b)

# FIG. 5

(a)

(b)

# FIG. 6

(a)

(b)

# FIG. 7

# FIG. 8

201

203

211
213
209
207
205

215

215
219
220
221
227
217
223
225

229
231
227
233

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004298384 A